(19) Europäisches Patentamt
European Patent Office
Office européen des brevets

(11) Numéro de publication: **0 131 500**
**B1**

(12) # FASCICULE DE BREVET EUROPÉEN

(45) Date de publication du fascicule du brevet: **20.05.87**

(21) Numéro de dépôt: **84401296.3**

(22) Date de dépôt: **21.06.84**

(51) Int. Cl.⁴: **C 07 C 153/09**, C 07 C 149/24, C 07 K 5/06, C 07 C 157/14, C 07 F 9/165, A 61 K 31/265, A 61 K 31/155, A 61 K 31/66, A 61 K 37/02

(54) **Nouveaux agents radioprotecteurs ayant une structure amino-thioalkyle et procédé pour leur préparation.**

(30) Priorité: **22.06.83 FR 8310318**

(43) Date de publication de la demande:
**16.01.85 Bulletin 85/3**

(45) Mention de la délivrance du brevet:
**20.05.87 Bulletin 87/21**

(84) Etats contractants désignés:
**AT BE CH DE FR GB IT LI LU NL SE**

(73) Titulaire: **CENTRE NATIONAL DE LA RECHERCHE SCIENTIFIQUE (CNRS), 15, Quai Anatole France, F-75700 Paris (FR)**

(72) Inventeur: **Oiry, Joel, 31 Lotissement Château Bon Route de Lavérune, F-34100 Montpellier (FR)**
Inventeur: **Imbach, Jean- Louis, Chemin Clos des Oliviers 1108 rue de Las Sorbes, F-34000 Montpellier (FR)**

(74) Mandataire: **Warcoin, Jacques, Cabinet Régimbeau 26, avenue Kléber, F-75116 Paris (FR)**

(56) Documents cité:
**GB-A-710 216**

**CHEMICAL ABSTRACTS, vol. 96, no. 21, 24 mai 1982, page 315, no. 176797m, Columbus, Ohio, US; E. SHAW et al.: "The specificity of cathepsin B"**
**CHEMICAL ABSTRACTS, vol. 67, no. 5, 31 juillet 1967, page 2036, no. 21570j, Columbus, Ohio, US; G.M. AIRAPETYAN et al.: "Preparation of some derivatives of beta-mercaptopropionic acid, cystamine, and cysteamine and study of their radoprotective activity"**
**CHEMICAL ABSTRACTS, vol. 61, no. 5, 31 août 1964, colonne 6081d, Columbus, Ohio, US; SHUICHI KIMURA et al.: "Biological activities of pantothenic acid analogs and Saccharomyces sake and lactic acid bacteria"**
**CHEMICAL ABSTRACTS, vol. 74, no. 23, 7 juin 1971, page 376, no. 124409n, Columbus, Ohio, US; F. YU**

(56) Documents cité: (suite)
**RACHINSKII et al.: "Chemical and physicochemical properties of aminothiols and amino disulfides. II. Stability of aminoalkane disulfides in an alkaline medium"**
**CHEMICAL ABSTRACTS, vol. 74, no. 5, 1 février 1971, page 79, no. 20105u, Columbus, Ohio, US; VILMOS VARTERESZ et al.: "Radioprotective effect of N-substituted AET (S-(2-aminoethyl) derivatives having amino acid structure"**

**EP 0 131 500 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

Les radioprotecteurs représentent une classe de composés particulièrement étudiés dans la littérature et caractérisés, pour la plupart d'entre eux, par la présence d'une fonction SH ou d'un de ses bioprécursueurs.

Ainsi, différentes publications traitent de certains de ces composés, en particulier le brevet GB-A-710 216 et les articles de E. SHAW et al. "The specificity of cathepsin B", (C.A. vol. 96 n° 21, 24 mai 1982, page 315, n°176797m, Columbus, Ohio, US et ACTA BIOL. MED. GER. 1981, 40 (10-11), 1503-11); de G.M AIRAPETYAN et al. "Preparation of some derivatives of beta-mercaptopropionic acid, cystamine, and cysteamine and study of their radioprotective activity", (C.A.vol. 67, n° 5, 31 juillet 1967, page 2036, n° 21570j, Columbus, Ohio, US et IZV. AKAD. NAUK SSSR, SER. KHIM. 1967 (2), 334-41); de SHUICHI KIMURA et al. "Biological activities of pantothenic acid analogs and Saccharomyces sake and lactic acid bacteria" (C.A.vol. 61, n° 5, 31 août 1964, colonne 6081d, Columbus, Ohio, US et J.VITAMINOL. (KYOTO) 9(3), 243-50 (1963) et de F. YU RACHINSKII et al. "Chemical and physicochemical properties of aminothiols and amino disulfides. II. Stability of aminoalkane disulfides in an alkaline medium", (C.A. vol. 74, n° 23, 7 juin 1971, page 376, n° 124409n, Columbus, Ohio, US et ZH. OBSHCH. KHIM. 1979, 40 (9), 2109-12).

Le principal mode d'action de ces composés est lié à la capacité présentée par le groupement thiol de piéger les radicaux libres induits intracellulairement par les radiations de diverse nature, empêchant ainsi des dommages permaments dans les cellules.

Une structure type, des plus étudiées, est celle de la cystéamine (ou MEA) (1):

$NH_2$-$CH_2$-$CH_2$-SH (1)

et le radioprotecteur actuellememt le plus actif est un dérivé de la cystéamine de structure (2): le WR 2721

$$NH_2-(CH_2)_3-NH-CH_2-CH_2-S-\overset{\overset{O}{\|}}{\underset{\underset{OH}{|}}{P}}-OH \qquad (2)$$

Ce composé est substitué du côté N terminal de la cystéamine par une chaîne aminoalkyle et du côté S terminal par une fonction phosphorothioate qui présente le double avantage de conferer une bonne solubilité aqueuse à la molécule et de protéger dans un premier stade la fonction thiol qui peut être liberée "in situ" par action des phosphatases ou même par hydrolyse.

En général, les radioprotecteurs conduisent à une protection non sélective des cellules, qu'elles soient saines ou malignes (par exemple la cystéamine, la cystéime, etc.).

Or, le WR 2721 possède la propriété remarquable de protéger sélectivement les tissus normaux contre les dommages induits par les radiations tout en laissant les tumeurs solides supporter seules l'effet des radiatioms.

En d'autres termes, cette drogue se concentre sélectivement dans les cellules saines et non dans les cellules cancéreuses. L'origine de cette sélectivité n'est pas encore fernememt établie.

Quoiqu'il en soit, l'intérêt de cette nouvelle classe de radioprotecteurs sélectifs est très importante de par leur utilisation potentielle en radiothérapie, ce qui permettrait de diminuer le nombre de cancers induits.

Il semblerait aussi que le WR 2721 puisse exercer un effet protecteur sélectif lors des chimiothérapies avec des drogues alkylantes telles que le cisplatine le cyclophosphamide et les moutardes à l'azote. De plus, ce composé est le premier radioprotecteur approuvé par la FDA et il a fait l'objet d'études phase I tant aux Etats-Unis d'Amerique qu'au Japon.

La présente invention concerne des dérivés de la cystéamine qui présentent un bon pouvoir radioprotecteur sélectif et dont certains sont des inducteurs d'interféron.

Les composés selon la présente invention correspondent à la formule:

$R_1$-CO-$NR_3$-/A/-S-$R_2$ (I)

dans lesquelles $R_1$ est un radical correspondant à un aminoacide $R_1$-COOH,

/A/est un radical alkylène en $C_2$ ou $C_3$ qui peut être substitué par un radical alcoyle en $C_1$ à $C_3$, un radical hydroxy libre, hydroxycarbonyle ou radical -COR dans lequel R est un radical amino ou -NH-$CH_2$-$CO_2$-H, $R_2$ est un radical protecteur de la fonction thiol qui libère la fonction thiol "in vivo", $R_3$ est l'hydrogène ou relié au groupement $R_2$, forme un cycle protecteur de la fonction thiol, à l'exception de la diglycyl-N,N'cystamine, de la di-β-alanyl-N,N'cystamine et de la di-phénylalanyl-N,N'-cystamine.

L'invention concerne également les sels de ces composés avec des acides pharmaceutiquement acceptables.

Parmi les aminoacides de formule $R_1$-COOH, il faut citer ceux dans lesquels $R_1$ est un radical alcoylamino en $C_1$ à $C_7$ droit ou ramifié, en particulier en $C_1$ à $C_4$ tel que les dérivés de l'alanine, de la glycine et de la sarcosine ainsi que les dérivés alkyles substitués sur l'amine. Le radical $R_1$ peut également provenir d'un di ou tripseudopeptide, par "pseudopeptide" on entend désigner un enchaînement de 2 ou 3 acides aminés naturels ou non, tels que ceux définis précédemment, par exemple un enchaînement gly-gly ou ala-gly.

$R_2$ est défini comme un radical protecteur de la fonction thiol, ceci est vrai au sens propre dans le cas des composés (I), mais pour les composés qui sont des dérivés symétriques de (I), il s'agit la d'une définition fonctionnelle puisque $R_2$ n'est pas sur un atome de soufre mais sur une fonction aminée, néanmoins les essais ont montré que les deux types de composés, présentaient des propriétés voisines.

Parmi les radicaux $R_2$, on peut envisager divers types de radicaux suivant la nature de la réaction qui va liberer la fonction thiol:

soit par transposition S → N,

par exemple:

$R_1$-CO-NH-$CH_2$-$CH_2$-S-$CR_5$(=$R_4$)→$R_1$-CO-N($R_4R_5$)-$CH_2$-$CH_2$-S-H

où $R_4$ = O, S, NH, N($C_1$-$C_7$)alkyle, $R_5$ = H, $C_1$-$C_7$ alkyle, aryle substitué ou non, $NH_2$, SH;

soit par une activation bioréductrice d'un pont disulfure, par exemple:

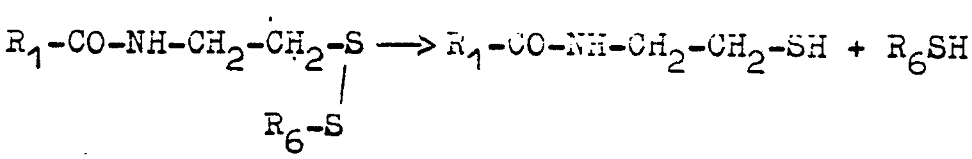

$R_6$ pouvant être une chaîne $R_7$-CO-$NR_3$-/A/, c'est-à-dire que le composé peut être un dérivé de la cystamine, ou bien un radical aryle ou $C_1$-$C_7$-alkyle non substitué ou substitué,

soit par hydrolyse enzymatique ou non, dans ce cas: $R_2$ = $SO_3H$ ou $PO_3H_2$ ou alcanoyloxy en $C_2$ à $C_7$;

enfin, le groupement thiol peut être masqué par inclusion dans une structure cyclique de type thiazolique, soit:

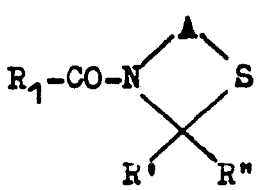

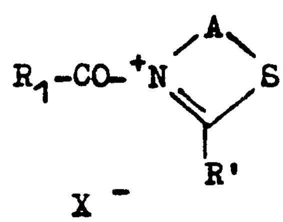

où R' = H, $C_1$-$C_7$-alkyle, aryle, $NH_2$,

R" = H, $C_1$-$C_7$-alkyle, aryle.

La plupart de ces composés possédant des atomes d'azote basique peuvent être préparés sous forme de sels quaternaires. L'anion utilisé $X^-$ étant de nature diverse, par exemple: $Br^-$, $Cl^-$, $CF_3CO_2^-$, $CH_3$-$C_6H_4$-$SO_3^-$, $CH_3CO_2^-$.

Parmi les radicaux asryles mentionnés, il faut citer les radicaux aryles monocycliques, notamment le radical phényle qui peut être substitue par un ou plusieurs atomes d'halogène notamment le chlore, par un ou plusieurs radicaux $C_1$-$C_3$-alkyle ou hydroxy.

Parmi les composés preférés de la présente invention, il faut citer les composés de formule:

$R_7$-NH-$alk_1$-(C=O)-NH-$alk_2$-S-(C=O)-$alk_3$

et

$R_7$-NH-$alk_1$-(C=O)-S-$alk_2$-NH-(C=O)-$alk_3$

dane lesquelles $R_7$ est H ou un radical alkyle en $C_1$ à $C_3$, $alk_1$ est une chaîne alkylène droite ou ramifiée en $C_1$ à $C_7$, $alk_2$ est une chaîne éthylényl ou propylényl portent un ou plusieurs radicaux méthyl ou hydroxy libre ou estérifié, $alk_3$ est un radical $C_1$-$C_7$-alkyle non substitué ou substitué par un ou plusieurs atomes de chlore, et notamment:

la glycyl-N-(S-acétyl)-cystéamime,

la L-alanyl-N-(S-acétyl)-cystéamine,

la γ-aminobutyryl-N-(S-acétyl)-cystéamine,

la glycol-N-(S-dichloroacétyl)-cystéamine,

le glycylamino-2-diméthyl-2,2-thioacétyl-1-éthane I 108,

le glycylamino-3-acétoxy-2-thioacétyl-1-propane I 111,

le glycyl-N[(méthyl-2-acétylmercapto-2-éthyl]-amide I 118,

la sarcosyl-N-(S-acétyl)-cystéamine I 123,

le glycylthio-2-diméthyl-1,1-acétamido-1-éthane I 107,

la glycyl-S-(N-acétyl)-cystéamine I 114.
ainsi que les sels à ammonium quaternaires correspondants,
ainsi que les composés de formule:

$$\begin{array}{l} R_8\text{-NH-alk}_1\text{-(C=O)-NH-alk}_2\text{-S} \\ \qquad\qquad\qquad\qquad\qquad\qquad\quad | \\ R_8\text{-NH-alk}_1\text{-(C=O)-NH-alk}_2\text{-S} \end{array}$$

dans laquelle $R_8$ est H ou un radical alkyle en $C_1$ à $C_3$, $alk_1$ est une chaîne alkylényl droite ou ramifiée en $C_1$ à $C_7$ et $alk_2$ est une chaîne éthylényl ou propylényl portant un ou plusieurs radicaux méthyl ou hydroxy libre ou estérifié, et notamment:
la diglycyl-N,N'-cystamine,
la di-L-alanyl-N,N'-cystamine,
le di-γ-aminobutyryl-N,N'-cystamine,
le di-(glycylamino-3-acétoxy-2-propyl)-1,2 disulfure I 110,
la di-sarcosyl-N,N'-cystamine I 124,
ainsi que les sels d'ammonium quaternaires correspondants.
Les composés des formules precédentes sont également intéressants lorsque $R_7$ ou $R_8$ représente le radical à un acide aminé ou d'un dipeptide, en particulier lorsque $R_7$ ou $R_8$ représente

$$-NH_2\text{-alk}_4\text{-}\underset{\underset{O}{\|}}{C}\text{-}$$

ou

$$NH_2\text{-alk}_5\text{-}\underset{\underset{O}{\|}}{C}\text{-NH-alk}_4\text{-}\underset{\underset{O}{\|}}{C}\text{-}$$

par exemple $alk_4$ et $alk_5$ étant des radicaux alkylène droit ou ramifié en $C_1$ à $C_7$ de préférence en $C_1$ à $C_4$.
Parmi ces composés il faut citer:
la di-glycylglycyl-N,N'-cystamine I 116,
la glycylglycyl-N-(S-acétyl)-cystésmine I 109.
Parmi les autres composés intéressants, on peut citer les composés de formule:
$R_7\text{-NH-alk}_1\text{-(C=O)-NH-alk}_2\text{-S-}R_9$
dans laquelle $R_7$, $alk_1$ et $alk_2$ ont les significations données précédemment et $R_9$ est

$$\underset{\underset{NH}{\|}}{C}\text{-NH}_2,\ -SO_3H \text{ ou } -PO_3H_2$$

ou les sels de ces fonctions.
En particulier on peut citer:
le glycyl-(N-isothiouronium-2-éthyl)amine
l'acide (glycylamino-2)-éthylthiosulfurique,
le glycylanino-2-S-éthylphosphorothioate de sodium.
L'un des avantages des composés selon l'invention par rapport su produit WR 2721 est qu'ils sont plus stables.
Les composés selon la presente invention peuvent être préparés par des procédés analogues à ceux utilisés

dans la synthèse des peptides.

Les significations des abréviations utilisées ont été rassemblées à la fin de la description.

De façon generale les composés selon l'invention sont obtenus par déprotection d'un composé de formule:

$(pro)R_1\text{-}CO\text{-}NR_3\text{-}[A]\text{-}S\text{-}R_2$

en éliminant le ou les groupements protecteurs (pro) du radical amino, les groupements protecteurs étant, de préférence les groupements benzyloxycarbonyl et terbutoxycarbonyl. Les composés protégés peuvent être synthétisés comme cela sera décrit ci-après.

Ainsi, les composés sont obtenus en couplant un AA N-protégé par un Z ou par un BOC avec une cystéamine (β-mercaptoathylamine-MEA) S-substituée (schéma 1). Les solvents de condensation sont l'acétate d'éthyle ou le THF. Le DCC ou le t-PNC (J. Martinez et coll., Bull. Soc. Chim. Fr. 1972, 12 4707) en présence de TEA sont les agents de couplage.

Les amines terminales sont ensuite déprotégées soit par l'acide bromhydrique en solution dans l'acide acétique glacial à basse température pour les Z, soit par l'acide trifluoroacétique pour les BOC et obtenues sous forme de bromhydrates ou de trifluoroacétates.

Le remplacement de l'aminoacide par un dipeptide ou tripeptide permet d'obtenir les mêmes dérivés S-substitués du schéma 1 en séries pseudo-dipeptidique ou tripeptidique etc. De même l'allongement de la chaîne côté N-terminal peut se faire par un nouveau couplage avec un autre amino-acide ou avec un dipeptide etc. correctement protégés ou activés.

Il est également possible de reproduire toutes les séquences qui précèdent mais en utilisant une halogénoalkylamine au lieu de la MEA-S-substituée. Les pseudopeptides halogénés, en particulier bromés, seront ensuite condensés avec les réactifs appropries pour obtenir les mêmes composés (schéma 2).

**Schéma 1**

Z ou BOC-AA + MEA S-substituée

AcOEt ou THF | DCC ou t-PNC/TEA

Z-AA-MEA S-substituée ou BOC-AA-MEA S-substituée

HBr/AcOH, Ø < -5°C | TFA/Thioanisole

HBr, AA-MEA S-substituée    TFA, AA-MEA S-substituée

AA peut être remplacé par AA-A'A'...etc...(AA=A'A'; AA≠A'A')

TFA, AA - MEA - S-Substituée + Z ou BOC-A'A'... / Z ou BOC-A'A'... ester actif

Z ou BOC ... A'A'-AA-MEA-S-Substituée

HBr ou TFA,...A'A'-AA-MEA-S-Substituée

Schéma 2

SCHEMA 2

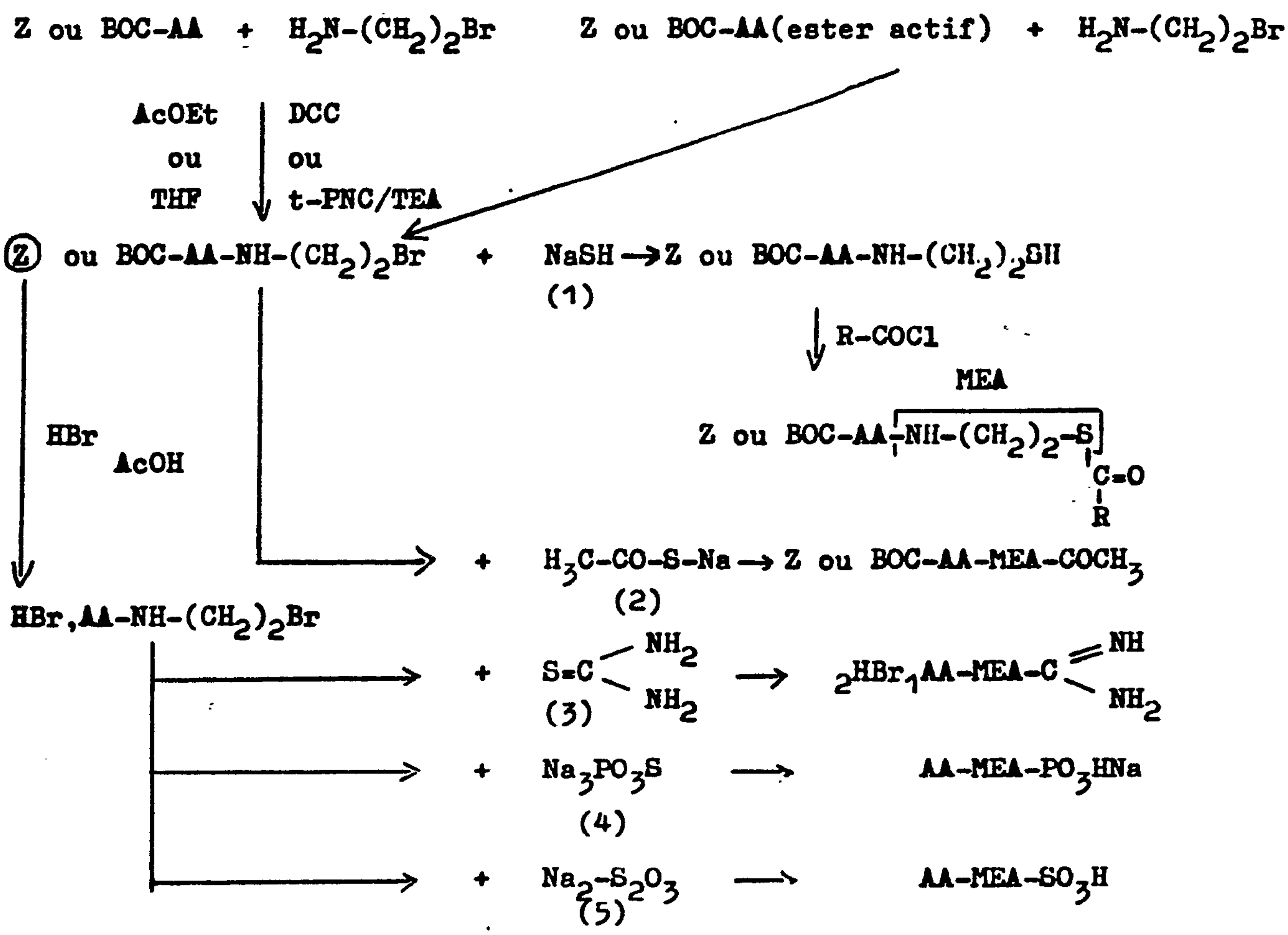


Il est également possible de parvenir aux mêmes S-substitutions de la façon suivante:

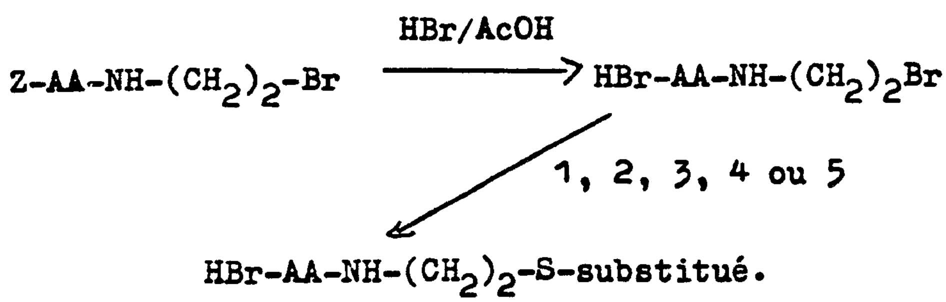


Parmi les esters actifs d'aminoacide utilisables dans ce type de réaction, il faut citer les composés de formule

$$\underbrace{Z-HN-(CH_2)_n-CO}_{AA}-\underbrace{O-R_7}_{\text{ester actif}}$$

$R_7$ = succinimido,
= para-nitrophényl,
= trichloro-2,4,5-phényl
= dichloro-2,6-p-nitrophényl.

La synthèse des dérivés de cystamine peut être réalisée selon deux méthodes différentes:

**Méthode utilisant les dérivés précédents de la MEA** (schéma 3)

Les deux pseudopeptides N-protégés par un Z ou un BOC et S-substitués sont hydrolysés par le méthylate de sodium côté S-terminal puis oxydés par l'iode dans de l'acide acétique aqueux pour former les Di-Z ou di-BOC pseudopeptides cystamines. Les amines terminales sont ensuite déprotégées par l'acide bromhydrique en solution acide acétique glacial ou par l'acide trifluoroacétique en présence de thioanisole. Les pseudopeptides sont obtenus sous forme de leurs di-bromhydrates ou di-trifluoroacétates.

Il est également possible d'éviter une étape en utilisant un dérivé halogéné (Z ou BOC) qui sera condensé avec l'hydrogénosulfure de sodium et formera directement le thiol correspondant Z-AA-MEA ou BOC-AA-MEA.

**Méthode utilisant la cystamine** (schéma 4)

Dans ce cas, on obtient directement des di-Z-AA-cystamine ou di-BOC-AA-cystamine. Ces dérivés sont synthétisés par condensation, dans le DMF en présence de TEA, d'un Z-AA activé ou d'un BOC-AA activé côté acide terminal par un ester approprié sur la cystamine.

Les di-Z ou di-BOC-AA-cystamine sont ensuite traités par l'acide bromhydrique en solution acide acétique glacial ou par l'acide trifluoroacétique et fournissent leurs sels d'amines respectifs.

les di-Z ou di-BOC-AA-cystamine sont également obtenus à partir des Z ou BOC glycines et de la cystamine en utilisant un agent de couplage (t-PNC ou DCC).

Les sels d'amines peuvent être libérés et remplacés par d'autres ions. De même la libération des sels des diaminoacide-cystamines permet d'augmenter la chaîne côté N-terminal avec d'autres aminoacides et de former des tétrapseudopeptides, etc., par couplages classiques. Il est également possible de les obtenir par les méthodes classiques déjà citées par couplage des Z ou BOC di, tripeptides, etc., activés ou non par un ester, avec la cystamine (schéma 5).

Schéma 3

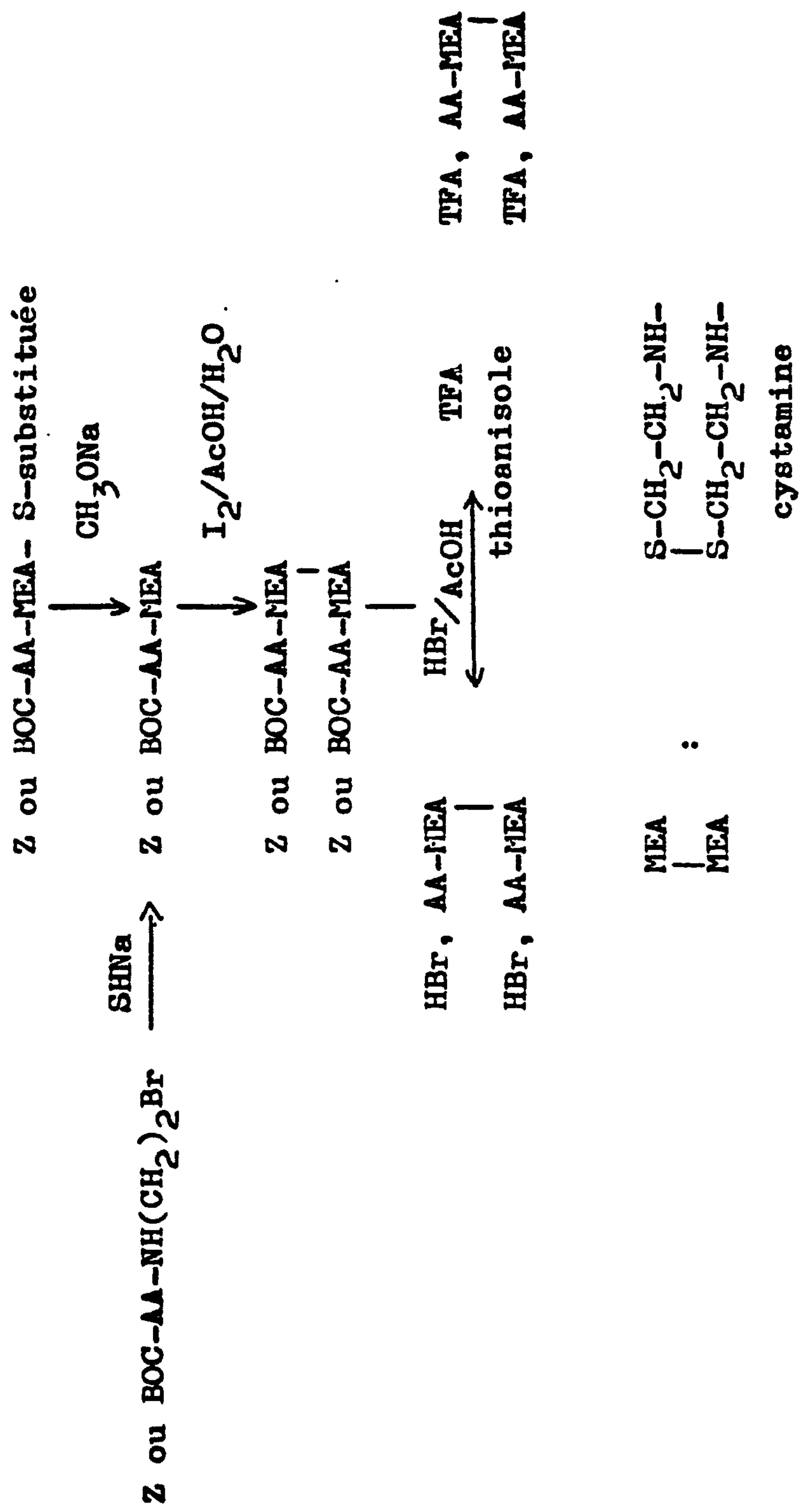

Schéma 4

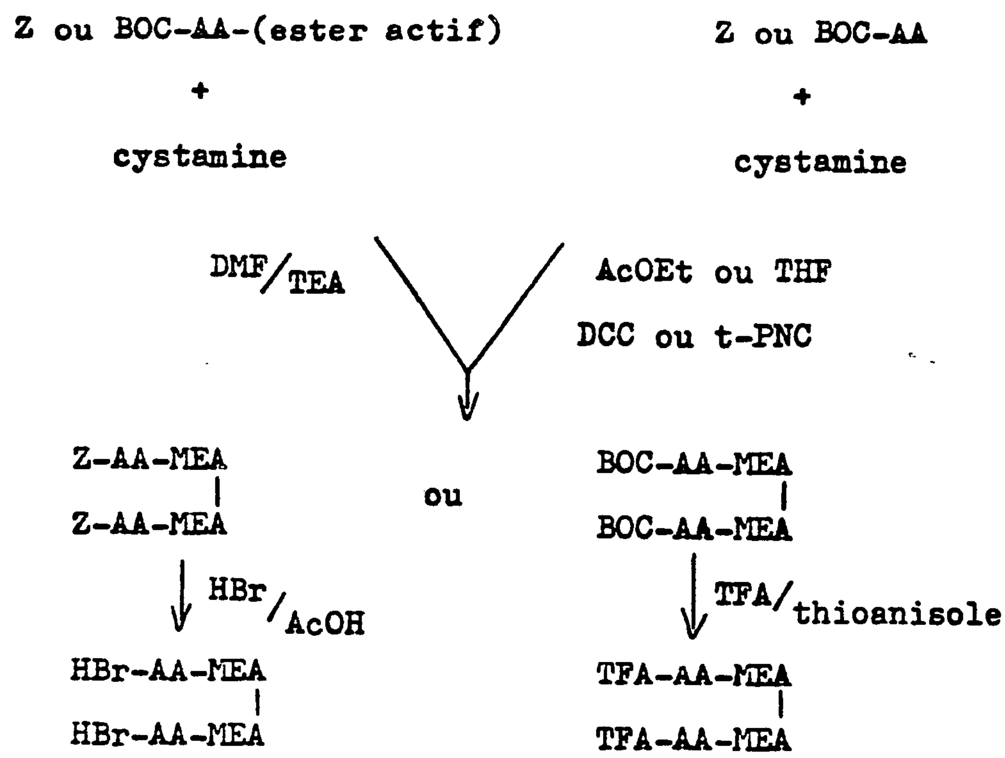


SCHEMA 5

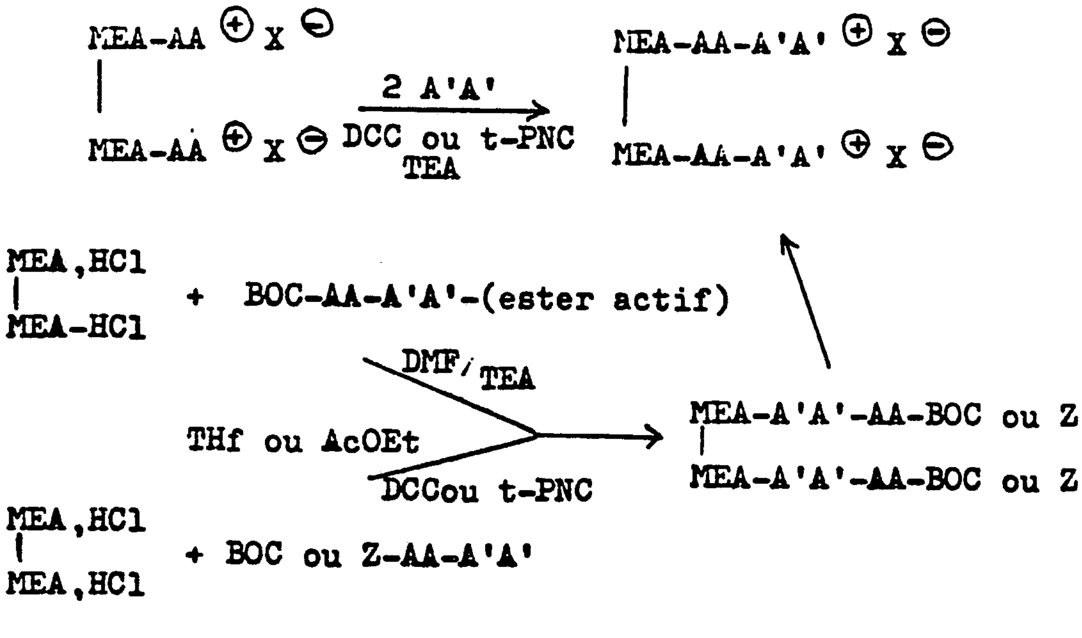


AA=A'A' ou AA ≠ AA'

La présente invention concerne également l'application des composés selon la présente invention à titre d'agent radioprotecteur et des compositions pharmaceutiques les contenant pour ce type d'application ou bien comme agent inducteur d'interféron dans le traitement des maladies sensibles à l'interféron.

D'autres caractéristiques et avantages de la présente invention apparaitront à la lecture des exemples ci-après.

**Exemple 1**

**Z-GLYCYL-N-(S-ACETYL)-CYSTEAMNINE. Méthode générale de couplage faisant intervenir un Z-AA**

Une solution de 4,598 g (0,022 mole) de Z-glycine dans 80 ml de THF est agitée à 0°C avec 3,88 g (0,011 mole) de t-PNC (le dichlohexylcarbodiimide-DCC également utilisé conduit au même rendement) préalablement dissous dans 20 ml de THP ou d'acétate d'éthyle. Au bout de 30 minutes d'agitation a 0°C, on ajoute 2,4 ml de TEA et on laisse agiter à nouveau 30 minutes. Au terme de ce temps on verse une solution de 3,421 g (0,022 mole) de chlorhydrate de S-acétyl-cystéamine (préparé selon T. Wieland et E. Bokelmann, Ann. Chem., 1952, 576, 20) dans 50 ml de THF ou d'acétate d'éthyle. L'agitation est maintenue à 0°C durant 30 minutes puis on laisse revenir à température ambiante en maintenant le pH basique par addition de TEA. La réaction est suivie par CCM dans un éluant de chloroforme/méthanol (9/1) et le temps nécessaire au couplage est d'environ 2-3 heures dans ce cas.

Le mélange est alors évaporé à sec sous pression réduite puis repris par 500 ml d'acétate d'éthyle et 2 x 200 ml de bicarbonate de sodium aqueux saturé glacé. La phase organique est ensuite décantée et lavée avec 2 x 200 ml d'acide chlorhydrique aqueux glacé 5 % et avec 2 x 200 ml d'eau (pH neutre) puis séchée sur sulfate de sodium et évaporée à sec sous vide. On recueille 5 g d'une huile légèrement jeune.

Le produit est purifié par chromatographie sur colonne de gel de silice (éluant: acétate d'éthyle/éther de pétrole 8/2).

CCM (chloroforme/méthanol 9/1) $R_F$ = 0,5. Détection: UV ou vapeurs d'iode, Rdt = 45 %. Cristallise de l'acétate d'éthyle. F = 100-103°C.

Analyse: $C_{14} H_{18} N_2 SO_4$ (310)
calculé %: C 54,19 H 5,80 N 9,03
trouvé %: C 54,28 H 5,68 N 9,10

Spectre IR (KBr) ν $cm^{-1}$ 3320, 3280 (NH); 1665, 1625 (C=O), 1515 (amide I)

Spectre de RMN (DMSO $d_6$): δ ppm 7,33 (s-5H) benzyl; 6,75 et 5,75 (2m-2H)2NH (échangeable à l'eau lourde); 5,10 (s-2H) $CH_2$ benzyl; 3,82 (d-2H) $CH_2$ CO multiplet entre 2,70 et 3,60 (4H) $CH_2$-$CH_2$; 2,30 (s-3H) $COCH_3$.

**Exemple 2**

**BROMHYDRATE DE GLYCYL-N-(S-ACETYL)-CYSTEAMINE. Méthode générale de déblocage de l'amine avec formation de bromhydrate**

On agite à une température inférieure à -5°C, sous atmosphère d'azote, en protégeant de la lumière, un mélange de 1,49 g ($4,8.10^{-3}$ mole) de Z-glycyl-N-(S-acétyl)-cystéamine et de 4 ml d'une solution fraîchement préparée d'acide acétique glacial (ACOH) saturé d'acide bromhydrique (HBr). En 30 minutes, la réaction est terminée. Le bromhydrate est precipité du mélange par addition de 80 ml d'éther anhydre glacé puis la phase éthérée est décantée. Cette opération de lavage à l'éther est effectuée 5 fois. L'huile résiduelle est ensuite reprise par 30 ml d'eau distillée et lyophilisée. On recueille avec un rendement de 90 % le dérivé attendu sous forme d'une pâte incolore et très hygroscopique. Il sera conservé sous atmosphère d'azote ou sous vide.

Spectre de RMN ($D_2O$): 3,78 (s-2H) $CH_2$ glycine; 3,46 (m-2H) N-$CH_2$; 3,05 (m-2H) S-$CH_2$; 2,35 (s-3H) $COCH_3$.

**Exemple 3**

**Z-L-ALANYL-N-(S-ACETYL-CYSTEAMINE. Réaction de couplage selon la méthode générale**

Réactifs utilisés: 11,25 g (0,005 mole) de Z-alanine dans 200 ml de THF. 8,7 g de t-PNC dans 20 ml de THF. 7 ml de TEA. 7,75 g (0,005 mole de chlorhydrate de S-acétyl-cystéamine dans 150 ml de THF.

Le mélange est maintenu basique par addition de 7 ml de TEA et la réaction suivie par CCM (éluant: dichlorométhane/méthanol 9/1) est terminée en 4 heures. Après les traitements déjà décrits, on obtient 15 g d'une huile qui est chromatographiée sur colonne de gel de silice (éluant: acétate d'éthyle/éther de pétrole 8/2). Le dérivé attendu est recueilli sous forme d'une huile qui cristallise dans un mélange d'acétate d'éthyle/éther de pétrole 6/3. Rdt = 80 %. F = 78-80°C.

Analyse: $C_{15} H_{20} N_2 SO_4$ (324)
calculé %: C 55,55 H 6,17 N 8,64
trouvé %: C 55,72 H 6,37 N 8,67

Spectre IR (KBr) ν $cm^{-1}$: 3320, 3280 (NH): 1662, 1620 (C=O); 1520 (amide I).

Spectre de RMN ($CDCl_3$) δ ppm: 7,36 (S-5H) benzyl; 7,0 (m-1H-échangeable à l'eau lourde) NH-$CH_2$; 5,90 (d-

1H) -échangeable à l'eau lourde- NH-CH; 5,11 (S-2H) $CH_2$ benzyl 4,23 (m-1H)-CH; 3,40 (m-2H) NH-$CH_2$; 3,0 (m-2H) S-$CH_2$; 2,30 (S-3H) CO-$CH_3$; 1,36 (d-3H) CH-$CH_3$.

**Exemple 4**

**BROMHYDRATE DE L-ALANYL N-(S-ACETYL)-CYSTEAMINE. Préparé selon la méthode générale décrite précédemment**

Réactifs utilisés: 1,418 g ($4,3.10^{-3}$ mole) de Z-alanyl-N-(S-acétyl)-cystéamine. 3 ml HBr/AcOH.

On recueille, après lyophilisation dans 8 ml d'eau, 1,19 g de bromhydrate (hygroscopique).

Spectre de RMN ($D_2O$) δ ppm: 4,10 (m-1H) CH; 3,50 (m-2H) N-$CH_2$; 3,10 (m-2H) S-$CH_2$; 2,38 (S-3H) $COCH_3$; 1,50 (d-3H) CH-$CH_3$.

**Exemple 5**

**Z- γ -AMINOBUTYRYL-N-(S-ACETYL)-CYSTEAMINE. Réaction de couplage selon la méthode générale**

Réactifs utilisés: 9,48 g (0,04 mole) d'acide Z-ν-aminobutyrique (préparé selon R.L. Evans et coll., J. Org. Chem., 1959, 24, 863-4) dans 75 ml d'acétate d'éthyle. 7,95 g (0,02 mole) de t-PNC dans 18 ml d'acétate d'éthyle. 8 ml de TEA. 6,22 g (0,04 mole) de chlorhydrate de S-acétyl-cystéamine dans 50 ml d'acétate d'éthyle.

Le mélange est maintenu basique par addition de 6 ml de TEA. La réaction suivie par CCM (éluant: dichlorométhane/méthanol 9,2/0,8) est terminée au bout de 20 heures. Après les differents traitements, on obtient 12,51 g d'une huile rosée qui est chromatographiée sur colonne de gel de silice (éluant: dichlorométhane/méthanol 96/4). On recueille 4,4 g de produit. CCM ($CH_2Cl_2$/MeOH 8 %). $R_F$: 0,45. Cristallise dans un mélange d'acétate d'éthyle/éther de pétrole ou de dichlorométhane/éther de pétrole. F = 100-101°C.

Analyse: $C_{16}H_{22}N_2SO_4$ (338)

calculé %: C 56,80 H 6,51 N 8,28

trouvé %: C 57,00 H 6,68 N 8,21

Spectre IR (KBr) ν $cm^{-1}$: 3320-3290 (NH); 1870 ($COCH_3$); 1625 (CONH); 1625 (amide I)

Spectre de RMN ($CDCl_3$) δ ppm: 7,31 (S-5H) benzyl; 6,30 et 5,32 (m-2H) NH; 5,10 (s-2H) $CH_2$ benzyl; 3,66 à 2,80 (m-6H) N-$CH_2$ et S-$CH_2$; 2,35 (s-3H) CO-$CH_3$; 2,30 à 1,60 (m-4H) $CH_2$-$CH_2$ de l'aminobutyryl.

**Exemple 6**

**BROMHYDRATE DE γ-AMINOBUTYRYL-N-(S-ACETYL)-CYSTEAMINE. Préparé selon la méthode génerale décrite précédemment**

Réactifs utilisés: 1,4 g ($4,1.10^{-3}$ mole) de Z- γ-aminobutyryl-N-(S-acétyl)-cystéamine. 3 ml HBr/AcOH.

On recueille après lyophilisation dans 5 ml d'eau, 1 g de bromhydrate hygroscopique.

Spectre de RMN ($D_2O$) δ ppm: 3,47 à 3,17 et 3,17 à 2,76 (2m-6H) N-$CH_2$ et S-$CH_2$; 2,50 à 1,66 (m-4H) $CH_2$-$CH_2$; 2,31 (S-3H) $COCH_3$.

**Exemple 7**

**BOC-GLYCYL-N-(S-ACETYL)-CYSTEAMINE. Méthode générale de couplage utilisant un BOC-AA**

Une solution de 9 g (0,05 mole) de BOC-glycine dans 150 ml d'acétate d'éthyle est agitée à 0°C avec 18 g (0,05 mole) de t-PNC (le DCC peut également remplacer le t-PNC) dans 100 ml d'acétate d'éthyle. Au bout de 30 minutes d'agitation à 0°C, on ajoute 7,25 ml de TEA et on laisse agiter à nouveau 15 minutes. Au terme de ce temps on verse dans le mélange 9,1 g (0,05 mole) de chlorhydrate de S-acétyl-cystéamine puis, goutte à goutte, 11 ml de TEA. La réaction suivie par CCM dans un mélange de dichlorométhane/méthanol 9/1 est complète en 5 heures.

Le mélange réactionnel est ensuite lavé à l'eau puis au bicarbonate de sodium aqueux saturé, glacé, à nouveau à l'eau, ensuite avec une solution aqueuse glacée d'acide citrique 1N et enfin à l'eau jusqu'à pH neutre. La phase organique est alors séchée sur sulfate de sodium et évaporée à sec sous vide. On recueille 12,5 g de brut sous forme d'une huile. Ce produit est purifié par chromatographie sur colonne de gel de silice (éluant: dichlorométhane/méthanol 9,4/0,6). CCM: (dichlorométhane/méthanol 9/1) $R_F$ = 0,8. Détection UV, vapeurs d'iode ou pulverisation d'une solution d'acide sulfurique à 10 % dans de l'éthanol suivie du chauffage de la plaque. Rdt. = 40 %. Cristaillise un mélange de dichlorométhane/éther de pétrole. F = 59-60°C.

Analyse: $C_{11}H_{20}N_2SO_4$ (276)

calculé %: C 47,82 H 7,24 N 10,14
trouve %: C 47,65 H 7,26 N 10,04
Spectre de RMN ($CDCl_3$) δ ppm: 7,20, 5,45 (2m-2H -échangeable a l'eau lourde-) 2NH; 4,07 (d-2H) $CH_2$ gly; 3,57 (m-2H) $NHCH_2$; 3,15 (m-2H) S-$CH_2$; 2,35 (S-3H) $COCH_3$; 1,47 (S-9H) terbutyl.

**Exemple 8**

**TRIFLUOROACETATE DE GLYCYL-N-(S-ACETYL)-CUSTEAMINE. Méthode générale de déprotection d'un BOC-AA avec formation de son trifluoroacétate correspondant**

En protégeant de l'humidité 2,03 g ($7.10^{-3}$ mole) de BOC-glycyl-N-(S-acétyl)-cystéamine sont agités à température ambiante avec 10 ml de TEA et 0,25 ml de thioanisole. La réaction suivie par CCM (éluant: dichlorométhane contenant 8 % de méthanol) est complète en 4 heures. Le trifluoroacétate est précipité du mélange sous forme huileuse par addition de 150 ml d'éther anhydre et lavé avec 2 x 150 ml d'éther puis séché dans un dessicateur sous vide contenant de l'anhydride phosphorique et de la potasse en plaques. Rendement pratiquement quantitatif. Une haute pureté est obtenue par dissolution du sel dans 10 ml d'eau distillée suivie d'une lyophilisation.

Etant hygroscopiques, ces sels sont en général conservés sous vide ou sous atmosphère d'azote. Néanmoins, pour ce composé il a été possible de le cristalliser dans un mélange d'acétate d'éthyle et d'éther de pétrole 3-1. F = 93-95°C. Spectre IR (KBr) γ $cm^{-1}$: plusieurs bandes indiquant la présence d'un sel d'amine, 3340, 3220, 3080, 2995, 2960, 2800, 2740, 2620, 2540; 1700, 1670 (C=O); 1590 (amide).

Spectre de RMN ($D_2O$) δ ppm: 3,70 (S-2H) $CH_2$ gly; 3,37 (t-2H) N-$CH_2$; 2,98 (t-2H) S-$CH_2$; 2,29 (S-3H) $COCH_3$.

**Exemple 9**

**BOC-GLYCYL-N-(S-DICHLOROACETYL)-CYSTEAMINE. Réaction de couplage selon la méthode générale des BOC-AA**

Réactifs utlisés; 8,69 g ($49.10^{-3}$ mole) de BOC-glycine dans 160 ml d'acétate d'éthyle. 17,3 g ($49.10^{-3}$ mole de t-PNC dans 50 ml d'acétate d'éthyle. 7 ml de TEA. 12,34 g ($55.10^{-3}$ mole) de chlorhydrate de S-dichloro-acétyl-cystéamine.

Le mélange est maintenu basique par addition goutte à goutte de 11 ml de TEA et la reaction suivie par CCM (éluant: dichlorométhane contenant 10 % de méthanol) est complète en 12 heures.

Après les traitements déjà décrits, on obtient 13 g d'un produit huileux qui est chromatographié sur colonne de gel de silice (solvant d'élution dichlorométhane contenant 3 % de méthanol). Le dérivé attendu est recueilli sous forme d'une huile qui cristallise dans un mélange de dichlorométhane/éther de pétrole 3/1. CCM (dichlorométhane/méthanol 9/1) $R_F$ = 0,85.

Analyse: $C_{10}\,H_{18}\,N_2\,SO_3\,Cl_2$ (317)
calculé %: 0 37,85 H 5,67 N 8,83
trouvé %: C 37,78 H 5,69 N 8,80
Spectre IR (KBr) ν $cm^{-1}$: 3320 (NH); 1680 (C=O); 1530 (amide I).
Spectre de RMN ($CDCl_3$) δ ppm: 7,22 et 5,47 (2m-2H) 2NH; 6,0 (S-1H) $CHCl_2$; 4,05 (d-2H) $CH_2$ gly; 3,54 (m-2H) N-$CH_2$; 3,13 (m-2H) S-$CH_2$; 1,45 (S-9H) terbutyl.

**Exemple 10**

**TRIFLUOROACETATE DE GLYCYL-N-(S-DICHLOROACETYL)-CYSTEAMINE. Méthode générale de déprotection d'un BOC (déjà décrite)**

Réactifs utilisés: 2,2 g ($69.10^{-4}$ mole) de BOC-glycyl-N-(S-dichloroacétyl)-cystéamine. 15 ml de TFA. 0,3 ml de thioanisole.

La réaction suivie par CCM (éluant: dichlorométhane contenant 10 % de méthanol) est complète en 3 heures. Après les differents lavages, on recueille 1,86 g (Rdt = 75 %) d'une huile incolore que l'on dissout dans 20 ml d'eau distillée et qu on lyophilise. Ce trifluoroacétate se présente alors sous forme d'une poudre hygroscopique.

Spectre de RMN ($D_2O$) δ ppm: 6,15 (S-1H) CH-$Cl_2$; 4,07 (S-2H) $CH_2$ gly; de 3,68 à 3,00 (2m-4H) N-$CH_2$-$CH_2$-S.

**Exemple 11**

**Z-GLYCYL-[N-(BROMO-2-ETHYL)]-AMIDE.**

**Première méthode** déjà décrite et qui fait intervenir un Z-AA en présence d'un agent de couplage et d'une amine. Réactifs utiliées: 5,2 g ($24.10^{-3}$ mole) de Z-glycine dans 50 ml d'acétate d'éthyle. 8,7 g ($25.10^{-3}$ mole) de t-PNC dans 25 ml d'acétate d'éthyle. 5 ml de TEA. 9,83 g ($48.10^{-3}$ mole) de bromhydrate de bromo-2-éthyl-amine (Fluka) que l'on libère dans l'acétate d'éthyle par la TEA avant de l'additionner au mélange réactionnel.

La réaction contrôlée par CCM (dichlorométhane contenant 5 % de méthanol) n'évolue plus au bout de 5 heures. On procède alors aux différents lavages et on obtient, après évaporation puis chromatographie sur colonne de gel de silice (éluant dichlorométhane contenant 1,5 % de méthanol) 3 g d'une huile jaunâtre qui cristallise dans un mélange de dichlorométhane/éther de pétrole. Rdt = 25 %. F = 111-113°C.

Analyse: $C_{12}H_{15}N_2BrO_3$ (315)
calculé %: C 45,71 H 4,76 N 8,88
trouvé %: C 45,68 H 4,77 N 8,92

Spectre IR (KBr) ν $cm^{-1}$: 3300 (NH); 1680, 1640 (C=0);
1530 (amide I).

Spectre de RMN ($CDCl_3$) δ ppm: 7,33 (S-5H) benzyl; 6,55 et 5,50 (2m-2H-échangeables à l'eau lourde-) 2NH; 5,10 (S-2H) $CH_2$ benzyl; 3,88 (d-2H) $CH_2$ gly; 3,65 (t-2H) $CH_2Br$; 3,42 (m-2H) N-$CH_2$.

**Deuxième méthode** (meilleurs rendements que la précédente)

Elle fait intervenir un Z-aminoacide activé.

Une solution agitée à 0°C de 6,146 g (0,03 mole) de bromhydrate de bromo-2-éthylamine dans 60 ml de DMF est additionnée goutte à goutte de 4,16 ml de TEA. Après l'addition de la base (20 minutes), on ajoute au mélange et toujours à 0°C 6,12 g (0,02 mole) de l'ester-Z-glycyl-O-N-succinimide, puis on laisse revenir à température ambiante. La réaction suivie par CCM (éluant dichlorométhane contenant 10 % de méthanol) est complète en 3 heures.

Le solvant est évaporé à sec sous vide et la pâte restante est reprise par 400 ml d'acétate d'éthyle. On procède ensuite aux différents lavages: 300 ml d'eau puis 300 ml de bicarbonate de sodium aqueux saturé et glacé, 300 ml d'eau, 300 ml d'acide chlorhydrique (0,1N) aqueux glacé et enfin à l'eau jusqu'à pH neutre. La phase organique est ensuite séchée sur sulfate de sodium et évaporée à sec sous vide. On recueille une poudre blanche qui cristallise dans un mélange de dichlorométhane/éther de pétrole. Rdt = 5 g, soit 79 %. Les données physico-chimiques de ce produit sont identiques aux précédentes.

**Exemple 12**

**BROMHYDRATE DE GLYCOL-(N-(BROMO-2-ETHYL))-AMIDE**

Sous atmosphère d'azote et en agitant, on traite à température ambiante 3,4 g ($10.10^{-3}$ mole) de Z-glycyl-(N-(bromo-2-éthyl))-amide par 9 ml d'acide acétique glacial saturé d'acide bromhydrique sec. La réaction suivie par CCM (éluant: dichlorométhane contenant 10 % de méthanol) ne contient plus de Z au bout de 30 minutes. Le bromhydrate est précipité du mélange sous forme d'une pâte par addition de 100 ml d'éther anhydre et le mélange est placé à 0°C durant 12 heures.

Les cristaux formée sont essorés et séchés dans un dessicateur sous vide sur anhydride phosphorique.

Rdt = 2,4 g soit 85,7 %. F = 158-160°C.

Spectre IR (KBr) ν $cm^{-1}$: 3400, 3200 ($NH_2$-NH); plusieurs bandes entre 3000 et 2580 indiquant la présence d'un sel 1660 (C=O); 1570 (amide I).

Spectre de RMN $D_2O$) δ ppm: 3,74 (S-2H) $CH_2$ gly; 3,70 à 3,33 (m-4H) $CH_2$-$CH_2$.

**Exemple 13**

**DIBROMHYDRATE DE GLYCYL [N-(ISOTHIOURONIUM-2-ETHYL)]-AMIDE**

Une solution de 1,31 g ($5.10^{-3}$ mole) de bromhydrate de glycyl[N-(bromo-2-éthyl)]-amide dans 10 ml d'éthanol absolu est additionnée de 0,380 g ($5.10^{-3}$ mole) de thiourée solubilisée dans 8 ml d'éthanol absolu. Le mélange est porte à ébullition durant 6 heures puis le solvant est concentré au 1/3 de son volume initial. Par refroidissement, il se forme des cristaux qui seront filtrés et recristallisés dans du méthanol. Rdt = 53 %. F = 178-180°C.

Spectre de RMN ($D_2O$) δ ppm; 3,80 (S-2H) $CH_2$ gly; 3,66 et 3,40 (2m-4H) $CH_2$-S et NH-$CH_2$.

**Exemple 14**

**Z-GLYCYL-N-CYSTEMAMINE**

(2 méthodes de synthèse)

**Première méthode**

Une solution de 2,5 g ($8.10^{-3}$ mole) de Z-glycyl-N-(S-acétyl)-cystéamine dans 40 ml de méthanol est traitée par 5 ml de méthylate de sodium (0,263 g de sodium dans 5 ml de méthanol). Le mélange réactionel est agité à température ambiante durant une heure puis amené à pH 1,2 par de l'acide chlorhydrique concentré. On évapore à sec sous vide la solution et on chromatographie sur gel de silice la pâte obtenue (éluant: dichlorométhane contenant 5 % de méthanol).

On recueille 1,5 g d'une huile incolore qui cristallise dans un mélange d'acétate d'éthyle et d'éther de pétrole. F = 95-96°C. CCM (chloroforme/méthanol 9,2/0,8) $R_F$ = 0,4 (détection à l'UV ou vapeurs d'iode).

Analyse: $C_{12}H_{16}N_2SO_3$ (268)

calculé %: C 53,73 H 5,97 N 10,45

trouvé %: C 53,70 H 5,86 N 10,35

Spectre de masse M+ 268

Spectre IR (KBr) ν $cm^{-1}$: 3320, 3280 (NH); 1680, 1650 (C=O); 1540 (amide I)

Spectre de RMN ($CDCl_3$) δ ppm: 7,36 (S-5H) benzyl; 6,83 (m-1H) NH (échangeables par addition de $D_2O$) cystéamine; 5,86 (t-1H) NH (échangeables par addition de $D_2O$) gly; 5,15 (S-2H) $CH_2$ benzyl; 3,86 (d-2H) $CH_2$ gly; 3,40 (m-2H) N-$CH_2$; 2,62 (m-2H) S-$CH_2$; 1,38 (t-1H) SH.

**Deuxième méthode**

L'action de l'hydrogénosulfure de sodium dans du méthanol sur le Z-glycol-[N-(bromo-2-étyl)]-amide conduit, après un reflux d'une heure suivi de l'évaporation du solvant et filtration de l'huile obtenue sur colonne de gel de silice, au même composé (données physico-chimiques identiques aux précédentes). Rdt = 74 %.

**Exemple 15**

**DI-Z-DIGLYCYL-N,N'-CYSTAMINE**

(3 méthodes de synthèse)

**Première méthode: oxydation du Z-gly-N-cystéamine**

Une solution agitée à température ambiante de 3,6 g ($13.10^{-3}$ mole) de Z-glycyl-N-cystéamine dans 15 ml d'acide acétique et 15 ml d'eau distillée est additionnée de 20 ml d'acide acétique contenant 250 mg d'iode, ce qui provoque le formation immédiete d'un précipité. Le mélange réactionnel est maintenu 15 minutes à température ambiante puis le précipité est filtré lavé à l'eau distillée puis séché dans un dessiceteur soue vide sur anhydride phosphorique. Recristallise dans un mélange de DMF et d'eau distillée. Les cristaux formés sont essorés puis laves avec un mélange d'éthanol et d'éther v/v, puis avec de l'éther. Après séchage sous vide sur anhydride phosphorique, on recueille avec un rendement de 40 % le dérivé attendu. F = 168-170°C. CCM (chloroforme contenant 10 % de méthanol) $R_F$ = 0,6.

Analyse: $C_{24}H_{30}N_4S_2O_6$ (534)

calculé %: C 53,93 H 5,61 N 10,48

trouvé %: C 54,14 H 5,73 N 10,50

Spectre de masse: M+: 534

Spectre IR (KBr) ν $cm^{-1}$: 3320 (NH) 1680, 1635 (C=O); 1535 (amide I)

Spectre de RMN ($DMSOd_6$) δ ppm: 8,12 et 7,44 (2m -échangeable à l'eau lourde-) 2xNH; 7,38 (S) benzyl; 5,05 (S) $CH_2$ benzyl; 3,62 (d) $CH_2$ gly; 3,50 à 3,10 et 3,10 à 2,58 (2m) N-$CH_2$-$CH_2$-S.

**Deuxième méthode: réaction entre la Z-glycine et la cystamine faisant intervenir un agent de couplage selon le méthode générale déjé décrite**

Réactifs utilisés: 7,2 g ($34.10^{-3}$ mole) de Z-gly dans 150 ml d'acétate d'éthyle. 12 g ($34.10^{-3}$ mole) de t-PNC (le DCC peut remplacer le t-PNC) dans 50 ml d'acétate d'éthyle. 5,1 ml de TEA. 3,04 g ($20.10^{-3}$ mole) de cystamine dans 30 ml d'acétate d'éthyle.

Lors de l'addition de le cystamine un précipité se forme instantanément. Le mélange réactionnel est agité à température ambiante durant 6 heures. Le précipité qui contient le dérivé attendu et du chlorhydrate de triéthylamine est essoré, lavé avec 100 ml d'acétate d'éthyle et 100 ml d'eau, puis recristallisé à environ 80°C dans du DMF contenant 10 % d'eau distillée. On recueille, après filtration et séchage dans un dessicateur sous vide sur anhydride phosphorique, 3,1 g de di-Z-diglycyl-N,N'-cystamine. Rdt = 34 % (critères physico-chimiques identiques aux précédents).

**Troisième méthode: selon une réaction déjà décrite et faisant intervenir un ester activé d'un AA N-protégé et une amine**

Réactifs utilisés: 2,252 g (0,01 mole) de di-chlorhydrate de cystamine dans 20 ml de DMF et 1,70 ml de TEA. 3,06 g (0,02 mole) d'ester Z-glycyl-O-N-succinimide dans 30 ml de DMF.

Le mélange réactionnel est agité 6 heures à température ambiante puis évaporé à sec sous vide. La pâte restante est triturée dans de l'acétate d'éthyle, ce qui provoque la formation d'une poudre incolore qui sera filtrée puis lavée à l'eau distillée. Le produit restant cristallise dans les mêmes conditions que précéderment.

Après filtration et séchage, on recueille 3,5 g d'un produit ayant des critéres physico-chimiques identiques aux précédents.

**Exemple 16**

**DIBROMHYDRATE DE DIGLYCYL-N,N'-CYSTAMINE. Réaction faite selon la méthode générale déjà décrite**

Réactifs utilisés: 1,8 g ($3.10^{-3}$ mole) de di-Z-diglycyl-N,N'-cystamine. 12 ml d'acide acétique glacial saturé d'acide bromhydrique, gazeux, sec.

Le mélange est agité 4 heures à température ambiante, puis on lui additionne, sous forte agitation, 100 ml d'éther anhydre ce qui favorise la formation d'une poudre légérement jaune. Cette poudre est essorée sous vide, puis lavée avec 2 x 50 ml d'éther ahydre et cristallisée dans un mélange de méthanol et d'éther.

On recueille, après cristallisation et séchage dans un dessicateur sous vide en présence d'anhydride phosphoriqus et de potasse 1,203 g (85 %) de dibromhydrate. F = 183-185° C.

Spectre de RMN($D_2O$) δ ppm: 3,78 (S) $CH_2$ gly; 3,55 (t) N-$CH_2$; 2,83 (t) S-$CH_2$.

**Exemple 17**

**DI-BOC-DIGLYCYL-N,N'-CYSTAMINE.**

(2 méthodes)

**Première méthode: section entre la BOC-glycine et la cystamine faisant intervenir un agent de couplage selon la méthode générale déjà décrite**

Réactifs utilisés: 2,98 g ($17.10^{-3}$ mole) de BOC-glycine dans 50 ml d'acétate d'éthyle. 5,92 g ($17.10^{-3}$ mole) de t-PNC (le DCC peut remplacer le t-PNC). 2,4 ml de TEA. 2,6 g ($17.10^{-3}$ mole) de cystamine dans 30 ml d'acétate d'éthyle.

La réaction contrôlée par CCM (éluant: dichlorométhane contenant 5 % de méthanol) est complète en 6 heures. On procède alors aux lavages classiques (eau, bicarbonate aqueux sature glacé, eau, acide citrique 1N glacé et eau jusqu'a pH neutre). La phase organique est séchée sur sulfate de sodium puis évaporée à sec sous vide. L'huile recueillie (impure) est chromatographiée sur colonne de gel de silice (gradient d'éluant d'une solution de dichlorométhane/méthanol allant de 2 à 5 % de méthanol). On recueille 3 g (Rdt = 37%) d'une huile incolore qui cristallise dans l'acétate d'éthyle. F = 97-98° C. $R_F$ = (dichlorométhane contenant 10% de méthanol) 0,4.

Spectre de RMN ($CDCl_3$) ppm : 7,24 (m-échangeable à l'eau lourde-) NH; 3,82 (d) $CH_2$ gly; 3,55 (m) N-$CH_2$; 2,77 (t) S-$CH_2$; 1,42 (S) ter-butyl.

**Deuxième méthode: réaction entre la BOC-glycine activée par un ester et la cystamine**

Réactifs utilises: 5,4 g ($24.10^{-3}$ mole) de dichlorhydrate de cystamine dans 50 ml de DMF 3,4 ml de TEA.

5,44 g ($20.10^{-3}$ mole) d'ester BOC-glycyl-O-N-succinimide.

Le mélange réactionnel est agité 6 heures à température ambiante et traité comme precédemment après évaporation a sec sous vide et reprise de la pâte obtenue par 500 ml d'acétate d'éthyle. Le produit brut obtenu cristallisé et homogène en CCM ne nécessite pas de chromatographie. Il sera directement recristallisé dans l'acétate d'éthyle. Rdt = 60 %. (données physico-chimiques identiques aux précédentes).

**Exemple 18**

**DI-TRIFLOUROACETATE DE DIGLYCOL-N,N'-CYSTAMINE. Réaction effectuée selon la méthode générale déjà décrite**

Réactifs utilisés: 1,7 g ($3{,}6.10^{-3}$ mole) de di-BOC-diglycyl-N,N'-cystamine. 6 ml de TFA. 0,06 ml de thioanisole.

Au bout de 3 heures d'agitation à température ambiante, on ne distingue plus de produit de départ sur la CCM. On procède alors aux traitements déjà décrits et on recueille, après lyophilisation, 1,79 g de produit sous forme huileuse (Rdt quantitatif).

Ce sel sera conservé comme les précédents sous atmosphère d'azote ou sous vide.

Spectre de RMN ($D_2O$) δ ppm: 3,61 (S) $CH_2$ gly; 3,38 (t) N-$CH_2$; 2,66 (t) S-$CH_2$.

**Exemple 19**

**DIBROMHYDRATE DE DIALANYL-N,N'-CYSTAMINE.**

La réaction est effectuée comme aux exemples 15 et 16 en remplaçant la Z-gly-N-cystéamine par la Z-als-N-cystéamine, les proportions molaires restant les mêmes.

Ce produit se présente après lyophilisation sous forme d'une huile.

Spectre de RMN ($D_{20}$): 4,10 (m) CH; 3,60 (m) N-$CH_2$; 2,90 (t) S-$CH_2$; 1,52 (d) $CH_3$.

**Exemples 20 et 21**

**BOC-L-ALANYL-N-(S-ACETYL)-CYSTAMINE.**

**1ère méthode**, faisant intervenir l'ester actif O-NSu de la BOC-L-alanine et réalisée selon la méthode générale précédemment décrite.

Réactifs utilisés: 21,5 g (75 x $10^{-3}$ mole) de BOC-L-Ala-ONSu, 200 ml de DMF, 11,6 g de chlorhydrate de S-acétylcystéamine, 10,45 ml de TEA (75 x $10^{-3}$ mole).

L'huile obtenue après les differents lavages est purifiée sur colonne de silice (éluant = acétonehexane 4/6). Cristallise dans un mélange d'éther et d'éther de pétrole 1/5. F = 71-73°C. CCM(ACOEt) Ef = 0,5. Rdt = 42,7 %.

Analyse: $C_{12} H_{22} N_2 O_4 S$ (290)
calculé %: C 49,65 H 7,58 N 9,65
trouvé %: C 49,61 H 7,60 N 9,62

Spectre de RMN ($CDCl_3$): 6,84 (t-1H) NH-$CH_2$; 5,25 (d-1H) NH-CH; 4,17 (m-1H)CH ala; 3,43 (m-2H) N-$CH_2$; 3,02(m-2H) S-$CH_2$; 2,33 (S-3H) CO-$CH_3$; 1,48 (S-9H) H terbutyl; 1,35 (d-3H) $CH_3$ ala.

**2ème méthode.**

Cette méthode est générale pour un couplage entre un aminoacide N-protégé et la cystéamine S-acétylée en présence d'un agent de couplage.

Une solution de 18,9 g (0,1 mole) de BOC-L-Ala. dans 200 ml d'acétate d'éthyle est agitée à 0°C avec 17,4 g (5 x $10^{-2}$ mole) de t-PNC préalablement dissous dans 100 ml d'acétate d'éthyle. Au bout de minutes d'agitation à 0°C, on sjoute 14 ml de TEA et on laisse à nouveau agiter 30 minutes. Au terme de ce temps, on additionne au mélange réactionnel 23,3 g (0,15 mole) de chlorhydrate de S-acétylcystéamine puis 21 ml de TEA.

La réaction est suivie par CCM dans un éluant de dichlorométhane-méthanol (9/1) et le temps nécessaire au couplage est d'environ 6 heures. On procède ensuite aux différents lavages (eau, bicarbonate de sodium aqueux glacé, eau, acide chlorhydrique 1 % glacé, eau jusqu'à neutralité. Après séchage de la phase organique sur sulfate de sodium et évaporation à sec sous vide on recueille 11,7 g d'une huile jaune. Cette huile est ensuite chromatographiée sur colonne de silice avec un éluant constitué de dichlorométnane et d'éther 8/2. On recueille 8,3 g de produit pur que l'on recristallise dans un mélange d'éther et d'éther de pétrole. Les données physico-chimiques de ce composé sont identiques aux précédentes (1ère méthode).

**Exemple 22**

**TRIFLUOROACETATE DE L-ALANYL-N-(S-ACETYL)-CYSTEAMINE: I 106**

La déprotection du BOC-L-alanyl-N-(S-acétyl)-cystéamine est réalisée selon la méthode générale. Réactifs utilisés: 5,7 g (19 x $10^{-3}$ mole) de BOC, 9,3 ml de TFA.

La réaction suivie par CCM est complète en 90 minutes. Après les différents lavages à l'éther le trifluoroacétate est repris par de l'eau distillée puis lyophilisé. On recueille 4,45 g d'une gomme.

Spectre de RMN($D_2O$) $\delta$ ppm: 3,95 (q-1H) CH ala; 3,40 (m-2H) N-$CH_2$; 2,98 (m-2H) S-$CH_2$; 2,31 (S-3H) CO-$CH_3$; 1,38 (d-3H) $CH_3$ ala.

**Exemple 23**

**BOC-GLYCYLTHIO-2-DIMETHYL-1,1-ACETAMIDO-1-ETHANE**

Le bromhydrate du thioacétamido-1 méthyl-2 amino-2 propane nécessaire pour les réactions suivantes est obtenu selon la méthode ci-dessous:

Une solution de 4,56 g (4 x $10^{-2}$ mole) de thioacétate de potassium dans 100 ml de DMF est agitée à 0°C durant 20 minutes et additionnée de 9,32 g (4 x $10^{-2}$ mole) de bromhydrate de bromo-1 méthyl-2 amino-2 propane (J.E. EARLEY et coll., J. Amer. Chem. Soc., 1958, 80, 3458). On laisse revenir la solution à température ambiante et l'agitation eat maintenue 12 heures. Le solvant est alors évaporé à sec sous vide à une température inférieure à 60°C. La pâte résiduelle est reprise par de l'acétonitrile ce qui précipite le bromure de potassium et le thioacétate de potaasium résiduel qui seront filtrés. Par évaporation des phases organiques on recueille 8,8

g de dérivé thioacétamide aous forme de gomme jaune. L'étude par RMN du proton de cette gomme révèle la présence d'environ 5% de dérivé bromé de départ; néanmoins elle sera utilisée impure pour la suite des réactions qui nécessitent des purifications par chromatographie ou recristallisation.

Deux méthodes sont possibles pour obtenir le BOC-glycylthio-2 diméthyl-1,1 acétamido-1 éthane:

**1ère méthode**, faisant intervenir l'ester actif O-NSu de la BOC-glycine et réalisée selon la méthode générale: Réactifs utilisés: 272 mg ($10^{-3}$ mole) de BOC-gly-ONSu, 10 ml de DMF, 280,1 mg ($1{,}23 \times 10^{-3}$ mole) de bromhydrate de thioacétamido-1 méthyl-2 amino-2 propane, 0,171 ml de TEA.

Après les différents traitements, on obtient le dérivé attendu avec un rendement de 40 %. Recristallise dans un mélange d'acétate d'éthyle et d'éther de pétrole. F = 134 - 136° C. CCM : Rf = 0,4 (acétate d'éthyle-éther de pétrole v/v : 8-2).

Analyse: $C_{13} H_{24} N_2 O_4 S$ (304)
calculé %: C 51,31 H 7,89 N 9,21
trouvé %: C 51,32 H 7,84 N 9,23

Spectre IR (KBr) $cm^{-1}$: 3340-3280 (NH); 3100-2980-2940 ($CH-CH_2-CH_3$); 1695-1685-1640 (C = 0); 1570-1535 (amide I).

Spectre de RMN ($CDCl_3$) δ ppm: 5,73 (S-1H) $NH-COCH_3$; 5,35 (m - 1H) NH gly; 4,10 (d-2H) $CH_2$) gly; 3,40 (S-2H) $S-CH_2$; 1,90 (S-3H) $CO-CH_3$; 1,48 (S-9H) H-terbutyl; 1,37 (S-6H) $C-(CH_3)_2$.

**2ème méthode**, faisant intervenir la glycine N-protégée et le bromhydrate de thioacétamido-1 méthyl-2 amino-2 propane en présence d'un agent de couplage (DCC). Réactifs utilisés: 6,75 g ($3{,}86 \times 10^{-2}$ mole) de BOC-gly, 40 ml de DMF, 8,8 g (≈ $3{,}8 \times 10^{-2}$ mole) de bromhydrate de thioacétamido-1 méthyl-2 amino-2 propane, 6,65 ml de DIEA, 7,95 g ($3{,}85 \times 10^{2}$ mole) de DCC. Temps réactionnel: 6 heures.

Après filtration de la DCU formée et évaporation de la DMF, la pâte obtenue est reprise par de l'acétate d'éthyle et on procède aux différents lavages. Après évaporation des phases organiques, on recueille 4,6 g d'un composé qui est en tous points identique au précédent (CCM-IR-RMN).

**Exemple 24**

**TRIFLUOROACETATE DE GLYCYLTHIO-2 DIMETHYL-1,1 ACETAMIDO-1 ETHANE: I 107**

La déprotection du BOC-glycylthio-2 diméthyl-1,1 acétamido-1 éthane eat réaliaée selon la méthode générale.

Réactifs utilisés: 1,9 g ($6{,}2 \times 10^{-3}$ mole) de BOC, 6 ml de TFA.

La réaction auivie par CCM (dichlorométhane-méthanol: v/v : 9-1) est totale en 90 minutes et le composé I 107, après avoir été précipité par addition d'éther anhydre, eat recriatallisé dans un mélange de méthanol et d'éther. On recueille 1,9 g de trifluoro-acétate. F = 165° C.

Spectre IR (kBr): ν $cm^{-1}$: 3300-3260 (NH); 3080-3000-2950 ($CH-CH_2-CH_3$); plusieurs bandes entre 2950 et 2500 en particulier 2740-2660 indiquent la présence du trifluoroacétate; 1690-1645 (C=O); 1565 (amide I).

Spectre RMN ($D_2O$) δ ppm: 4,13 (S-2H) $CH_2$ gly; 3,50 (S-2H) $S-CH_2$; 1,88 (S-3H) $CO-CH_3$; 1,29 (S-6H) $C-(CH_3)_2$.

**Exemple 25**

**BOC-GLYCYLAMINO-2 DIMETHYL-2,2 THIOACETHYL-1 ETHANE**

La synthèse de ce composé est réalisée selon la méthode générale faisant intervenir la glycine N-protégée et le bromhydrate de thioacétamido-1 méthyl-2 amino-2 propane en présence d'un agent de couplage (t-PNC).

Réactifs utilisés: 4,53 g (2,5 g x $10^{-2}$ mole) de BOC-gly, 50 ml d'acétate d'éthyle, 4,51 g ($12{,}9 \times 10^{-3}$ mole) de t-PNC, 3,61 ml de TEA, 5,9 g ($2{,}59 \times 10^{-2}$ mole) de bromhydrate de thioacétamido-1 méthyl-2 amino-2 propane, 3,61 ml de TEA.

La réaction suivie par CCM est totale en 8 heures. Le lavages classiques sont alors réalisés et après évaporation des phases organiques, on recueille un produit huileux qui est purifié par chromatographie sur colonne de silice en utilisant un mélange d'éluants constitué d'acétate d'éthyle et de dichlorométhane v/v: 6-4. A nouveau le produit purifié se présente sous forme huileuse (≈ 2 g). CCM ($CH_2Cl_2$-MeOH v/v: 4-6) Rf = 0,6.

Analyse $C_{13}H_{24}N_2O_4S$ (304)
calculé %: C 51,31 H 7,89 N 9,21.
trouvé %: C 51,29 H 7,90 N 9,24.

Spectre de RMN ($CDCl_3$) δ ppm: 6,57 (S-1H) NH-C-; 5,70 (m-1H) NH gly; 3,66 (m-2H) $CH_2$ gly; 3,34 (S-2H) $S-CH_2$; 2,33 (S-3H) $CO-CH_3$; 1,50 à 1,10 (m-15H) $C-(CH_3)_2$ et H-terbutyl.

**Exemple 26**

**TRIFLUOROACETATE DE GLYCYLAMINO-2 DIMETHYL-2,2 THIOACETYL-1 ETHANE: I 108**

La déprotection du BOC-glycylamino-2 diméthyl-2,2 thioacétyl-1 éthane est réalisée selon la méthode générale:

Réactifs utilisés: 1,9 g (6,2 x $10^{-3}$ mole) de BOC-glycylamino-2 diméthyl-2,2 thioacétyl-1 éthane, 6 ml de TFA.

La réaction suivie par CCM est totale en 60 minutes et le compose I 108 ne cristallisant pas après les traitements à l'éther anhydre sera repris par de l'eau distillée puis lyophilisé. On recueille 1,8 g d'une huile légèrement jaune.

Spectre de RMN ($D_2O$) δ ppm: 3,50 (S-2H) $CH_2$ gly; 3,16 (S-2H) S-$CH_2$; 2,19 (S-3H) CO-$CH_3$; 1,13 (S-6H) C-$(CH_3)_2$.

**Exemple 27**

**BOC-GLYCYLGLYCYL-N-(S-ACETYL)-CYSTEAMINE**

L'obtention de ce composé est réalisée selon la méthode générale faisant intervenir la glycylglycine N protegée et le chlorhydrate de S-acétylcystéamine en présence du t-PNC comme agent de couplage.

Réactifs utilisés: 7 g (3,02 x $10^{-2}$ mole) de BOC-glygly, 70 ml de THF, 10,5 g (3,02 x $10^{-2}$ mole) de t-PNC, 4,21 ml (3,02 x $10^{-2}$ mole) de TEA, 4,7 g (3,02 x $10^{-2}$ mole) de chlorhydrate de S-acétylcystéamine.

La réaction est traitée après 8 heures d'agitation. Le THF est évaporé à sec sous pression réduite. Le résidu est repris par de l'acétate d'éthyle et on procède aux differents lavages. Après évaporation des phases organiques on recueille 12 g d'un mélange huileux impur en CCM. Le produit de couplage est isolé pur après deux chromatographies sur colonne de gel de silice en utilisant de l'acétate d'éthyle comme éluant. On recueille 2,5 g (24 8 %) d'une huile qui cristallise dans un mélange d'acétate d'éthyle et d'éther de pétrole. F = 109-111°C. CCM ($CH_2Cl_2$-MeOH v/v: 9:1) Rf = 0,3.

Analyse: $C_{13}$ $H_{23}$ $N_3$ $O_5$ S (333),

calculé %: C 46,84 H 6,90 N12, 61

trouvé %: C 46,84 H 6,92 N 12,58.

Spectre IR (KBr) ν $cm^{-1}$: 3290 (NH); 3070-2980-2950 (CH-$CH_2$-$CH_3$); 1680-1640 (C=O); 1550 (amide I).

Spectre de RMN ($CDCl_3$) δ ppm: 7,33 (m-2H) 2NH gly; 5,80 (m-1H) NH(cystéamine); 3,88 (dd-4H) $CH_2$ gly; 3,35 (m-2H) N-$CH_2$ (cystéamine); 2,98 (m-2H) S-$CH_2$; 2,34 (S-3H) CO-$CH_3$; 1,44 (S-9H)H-tertbutyl.

**Exemple 28**

**TRIFLUOROACETATE DE GLYCYLGLYCYL-N-(S-ACETYL)-CYSTEAMINE I 109**

La déprotection du BOC-glycylglycyl-N-(S-acéthyl)-cystéamine est réalisée selon la méthode générale:

Réactifs utilisés: 1,05 g (3,15 x $10^{-3}$ mole) de BOC, 6 ml de TFA.

La réaction suivie par CCM est complète en 90 minutes et le composé I 109, après avoir été précipité par addition d'éther anhydre, est recristallisé dans un mélange de méthanol et d'éther. On recueille 1,100 g de trifluoroacétate, F = 117-118°C.

Spectre IR (KBr) ν $cm^{-1}$: 3300 (NH); 3130-3090-2960 (CH-$CH_2$-$CH_3$); plusieurs bandes entre 2980 et 2350 en particulier 2880-2760-2600-2370 indiquent la présence du triflouroacétate; 1680-1650 (C=O); 1540 (amide I).

Spectre de RMN ($D_2O$) δ ppm: 3,78 et 3,75 (2S-4H) $CH_2$ gly; 3,26 (m-2H) N-$CH_2$; 2,90 (m-2H) S-$CH_2$; 2,23 (S-3H) CO-$CH_3$.

**Exemple 29**

**BOC-GLYCYLAMINO-3 BROMO-1 PROPANOL-2**

Il est obtenu selon la méthode générale et il fait intervenir l'ester O-NSu de la glycine N protégée ainsi que le bromhydrete de bromo-1 amino-3 propanol-2 décrit par D.M. BALL et Coll., J. Org. Chem. 1963, 28, 1580.

Réactifs utilisés: 9,21 g (3,38 x $10^{-2}$ mole) de BOC-gly-ONSu, 100 ml de DMF, 7,95 g (3,38 x $10^{-2}$ mole) de bromhydrate de bromo-1 amino-3 propanol-2, 4,75 ml (3,38 x $10^{-2}$ mole) de TEA.

La réaction suivie par CCM est complète en 6 heures. Le DMF est alors évaporé à sec sous vide. La gomme restante est reprise par de l'acétate d'éthyle et on procède aux différents lavages. Après évaporation des solvants, on recueille 6,20 g d'une huile qui recristallise dans un mélange d'acétate d'éthyle et d'éther de pétrole. F = 94-96°C. CCM ($CH_2Cl_2$-MeOH v/v 9-1) Rf = 0,5 (détection aux vapeurs d'iode ou chauffage de la plaque-température > 200°C).

Analyse: $C_{10}$ $H_{19}$ $N_2$ $BrO_4$ (311)

Calculé %: C 38,58 H 6,10 N 9,00

Trouvé %: C 38,61 H 6,08 N 9,03

Spectre IR(KBr) ν $cm^{-1}$ 3380 (NH-OH) bande large; 2980-2940 ($CH-CH_2$); 1690 (C=O); 1525 (amide I). Spectre de RMN ($CDCl_3$) δ ppm: 7,10 et 5,66 (2m-2H), 2NH; 4,45 à 3,30 (3m-8H) $CH_2$ gly, $CH_2$-CH, CH-OH, $CH_2Br$; 1,47 (S-9H)H-terbutyl

**Exemple 30**

**BOC-GLYCYLAMINO-3 ACETOXY-2 THIOACETYL-1 PROPANE et DI-(BOC-GLYCYLAMINO-3 ACETOXY-2 PROPYL)-1,2 DISULFURE**

Une solution agitée de 1,01 g (8,8 x $10^{-3}$ mole) de thioacétate de potassium dans 20 ml de DMF est agitée à 0°C durant 15 minutes et additionnée de 2,10 g (6,7 x $10^{-3}$ mole) de BOC-glycylamino-3 bromo-1 propanol-2. Le mélange réactionnel est maintenu à 0°C durant 2 heures puis abandonné 10 heures à la température ambiante. Le solvant est ensuite évaporé à sec sous vide et la pâte restante est coévaporée avec de l'éther de pétrole. Par CCM avec une double migration dans l'éther on note la présence de plusieurs spots en particulier les deux produits attendus et un troisième qui est positif au test du nitroprussiate de sodium.

Il s'agit donc d'un dérivé contenant un thiol. Les trois produits étant difficilement séparables par chromatographie sur colonne de silice nous avons soumis toute la pâte obtenue précédemment à une oxydation dans du méthanol en présence d'iode et d'acétate de sodium. L'iode est additionné jusqu'a coloration persistante de la solution. La CCM de ce milieu ne montre plus que deux spots negatifs au test des SH.

Le mélange est alors évaporé à sec sous vide, repris par de l'acétate d'éthyle et lavé au thiosulfate de sodium puis à l'eau. Les phases organiques sont rassemblées séchées sur sulfate de sodium et évaporées à sec sous vide. On recueille environ 2 g d'une huile incolore qui sera chromatographiée sur colonne de silice avec un éluant constitué d'un mélange de dichlorométhane et d'éther v/v: 6-4.

Le composé le moins polaire en CCM (éluant $CH_2Cl_2$-MeOH v/v: 9-1) Rf = 0,8 a été identifié comme étant le BOC-glycylamino-3 acétoxy-2 thioacétyl-1 propane. Isolé sous forme huileuse ce produit cristallise dans un mélange d'acétate d'éthyle et d'hexane. F = 74-76°C.

Analyse: $C_{14}H_{24}N_2O_6S$ (348)

Calculé %: C 48,27 H 6,89 N 8,04

Trouvé %: C 48,29 H 6,85 N 8,07

Spectre IR(KBr) ν $cm^{-1}$ 3330-3260 (NH); 3080-2980-2940 ($CH-CH_2-CH_3$); 1740-1690-1650 (C = O) 1520-1510 (amide I).

Spectre de RMN ($CDCl_3$) δ ppm: 6,93 et 5,53 (2m-2H) 2NH; 4,98(m-1H) CH; 3,80 (d-2H) $CH_2$ gly; 3,48 (m-2H) N-$CH_2$; 3,13 (m-2H) S-$CH_2$; 2,33 (S-3H) S-$COCH_3$; 2,07 (S-3H) O-CO-$CH_3$; 1,48 (S-9H) H-terbutyl.

Le composé le plus polaire en CCM (même éluant que précédemment) a un Rf de 0,75 et a été identifié comme étant le di-(BOC-glycylamino-3 acétoxy-2 propyl)-1,2 disulfure. Ce produit a été obtenu sous forme huileuse.

Analyse: $C_{24}H_{42}N_4O_{10}S_2$ (610)

Calculé: C 47,21 H 6,88 N 9,18

Trouvé %: C 47,23 H 6,84 N 9,22

Spectre de RMN ($CDCl_3$) δ ppm: 7,12 et 5,80 (2m-4H)NH; 5,16 (m-2H) CH; 3,80 (d-4H) $CH_2$ gly; 3,56 (m-4H) N-$CH_2$; 2,93 (d-4H) S-$CH_2$; 2,06 (S-6H) O-CO-$CH_3$; 1,33 (S-18H) H-terbutyl

Un échantillon du dérivé présentant un thiol libre a pu être isolé du melange, avant l'oxydation par l'iode et identifié. Il s'agit bien du BOC-glycyl-amino-3 acétoxy-2 propanethiol-1.

Spectre de RMN ($CDCl_3$) δ ppm: 7,13 et 5,84 (2m-2H) NH; 4,90 (m-1H) CH; 3,80 (d-2H) $CH_2$ gly.; 3,59 (m-2H) N-$CH_2$; 2,72 (m-2H) S-$CH_2$ 2,06 (S-3H)O-CO-$CH_3$; 1,70 (t-1H) SH; 1,46 (S-9H) H-terbutyl.

**Exemple 31**

**DI-TRIFLUOROACETATE DE DI-(GLYCYLAMINO-3 ACETOXY-2 PROPYL)-1,2 DISULFURE: I 110**

La déprotection du di-(BOC-glycylamino-3 acétoxy-2 propyl)-1,2 disulfure est réalisée selon la méthode générale.

Réactifs utilises 940 mg (1,5 x $10^{-3}$ moles) de di-(BOC-glycylamino-3 acétoxy-2 propyl)-1,2 disulfure; 8 ml de TFA.

La réaction suivie par CCM est totale en 90 minutes et le composé ne cristallisant pas après les lavages à l'éther anhydre sera lyophilisé. On recueille 859,4 mg d'une huile (Rdt = 87,4 %).

Spectre de RMN ($D_2O$) δ ppm: 4,96 (m-2H) CH; 3,60 (S-4H) $CH_2$ gly; 3,33(m-4H)N-$CH_2$; 2,74(d-4H)S-$CH_2$ 1 88(S-6H)O-CO-$CH_3$.

**EXEMPLE 32**

**TRIFLUOROACETATE DE GLYCYLAMINO-3 ACETOXY-2 THIOACETYL 1 PROPANE : I 111**

La déprotection du BOC-glycylamino-3 acétoxy-2 thioacetyl-1 propane est réalisée selon la méthode générale.

Réactifs utilisés 1,4 g (4 x $10^{--3}$ mole) de BOC-glycylamino-3 acétoxy-2 thioacétyl-1 propane, 6 ml de TFA.

La réaction suivie par CCM est totale en 90 minutes et le composé I 111 ne cristallisant pas après les lavages a l'éther anhydre sera lyophilisé. On recueille 1,383 g d'une huile (Rdt = 95,5 %). Spectre de RMN ($D_2O$) o ppm 5,01 (m-1H)CH; 3,80(S-2H) $CH_2$ gly: 3,49(m-2H)N-$CH_2$, 3,13(m-2H)S-$CH_2$; 2,28(S-3H)S-$COCH_3$; 2 08 (S-3H)O-CO-$CH_3$.

**EXEMPLE 33**

**BOC-GLYCYL-S-(N-ACETYL)-CYSTEAMINE**

Une solution contenant 7 g (4 x $10^{-2}$ mole) de BOC-glycine dans 60 ml de DMF est agitée et refroidie à 0°C puis additionnée de 8,24 g (4 x $10^{-2}$ mole) de DCC. On verse ensuite sur le mélange à 0°C 6,22 g (4 x $10^{-2}$ mole) de chlorhydrate de S-acétylcystéamine puis goutte à goutte une solution de 5,56 ml de TEA dans 20 ml de DMF. Lorsque la TEA est additionnée, on laisse le mélange revenir à la température ambiante et on poursuit l'agitation 12 heures. Le précipité de DCU formé est essoré et le filtrat est concentré à sec aous vide. La pâte résiduelle est reprise par de l'acétate d'ethyle et on procède aux différents lavages de la solution eau; bicarbonate de sodium aqueux saturé et glacé; eau; acide citrique aqueux 1N; eau jusqu'à neutralité. Les phases organiques sont rassemblées, sécheés sur sulfate de sodium et évaporées a sec sous vide. On recueille une huile impure en CCM (éluant : acétate d'éthyle). Deux spots à Rf 0,4 et 0,25 sont prédominants. Les deux produits séparés par chromatographie sur colonne de silice (éluant: $CH_2Cl_2$ contenant 2% de methanol). Le composé le moins polaire (Rf 0,4), 3 g, à été isolé et identifié au BOC-glycyl-N-(S-acétyl)-cystéamine.

Le composé le plus polaire (Rf 0,25) a été recueilli sous forme d'une huile (2,55 g) qui recristallise dans un mélange d'acétate d'éthyle et d'éther de pétrole. F = 120°C.

Analyse $C_{11}$ $H_{20}$ $N_2$ S $O_4$ (276)

Calculé % : C 47,82 H 7,24 N 10,14

Trouvé % : C 47,79 H 7,26 N 10,16

Spectre IR (KBr) ν $cm^{-1}$ 3340-3260 (NH); 3060-3000-2960 (CH-$CH_2$-$CH_3$); 1695-1660 (C = 0); 1550 (amide I). Spectre RMN ($CDCl_3$) δ ppm: 7,22 et 6,28 (2m-2H)NH; 4,14(d-2H)$CH_2$ gly; 3,48(m-2H)N-$CH_2$; 3,26 (m-2H)-S-$CH_2$; 2,10 (S-3H)CO-$CH_3$; 1,58 (S-9H)H-terbutyl.

**EXEMPLE 34**

**TRIFLUOROACETATE DE GLYCYL-S-(N-ACETYL)-CYSTEAMINE: I 114**

Le déprotection du BOC-glycyl-S-(N-acétyl)-cystéamine est réalisée selon la méthode générale.

Réactifs utilisés 2,072 g (7,5 x $10^{-3}$ mole) de BOC. 3 ml de TFA.

La réaction suivie par CCM est complète en 2 heures et le composé I 114 après avoir été lavé avec de l'éther anhydre, est repris par de l'eau distillée puis lyophilisé. On recueille 2,1 g d'une huile jaune. Rdt = 96 %.

Spectre de RMN ($D_20$) δ ppm: 4, 12(S-2H)$CH_2$ gly; de 3,58 à 2,83 (m-4H)S-$CH_2$-$CH_2$-N; 1,92 (S-3H)CO-$CH_3$.

**EXEMPLE 35**

**DI-BOC-DI-GLYCYLGLYCYL-N,N'-CYSTAMINE**

Des trois méthodes envisagées et realisées, sur le schéma 5 nous ne décrirons que celle nous ayant fourni le composé avec les meilleurs rendements selon la méthode générale qui fait intervenir un ester activé d'un dipeptide et la cystamine.

Réactifs utilisés: 1,194 g (5,3 x $10^{-3}$ mole) de dichlorhydrate de cystamine dans 40 ml de DMF et 1,48 ml de TEA. 3,5 g d'ester Boc-glycylglycyl-O-N-succinimide.

Le mélange réactionnel est agité 12 heures à température ambiante puis évaporé à sec sous vide. La pâte restante est reprise par de l'acétate d'éthyle. On procède alors aux lavages classiques (eau, bicarbonate de sodium aqueux saturé glacé, eau, HCl 1 %, eau jusqu'à pH neutre). La phase organique est séchée sur sulfate de sodium puis évaporée à sec sous vide. On recueille 2,3 g d'une poudre incolore - qui recristallise dans un mélange d'acétate d'éthyle et d'éther de petrole. F = 118-120°C. RF = (Butanol-éthanol-eauv/v/v 2-1-1.) 0,8.

Analyse $C_{22}$ $H_{40}$ $N_6$ $S_2$ $O_8$ (580)

Calculé % C 45,51 H 6,89 N 14,48

Trouvé % C 45,49 H 6,91 N 14 48

Spectre de RMN (DMSO) δ ppm; 7,40 et 5,80 (2m-6H) 6NH; 4,0 (2d-8H)$CH_2$ gly; 3,53 (m-4H) N-$CH_2$; 2,95 (m-4H) S-$CH_2$; 1,44 (S-18H) terbutyl.

**EXEMPLE 36**

**DI-TRIFLUOROACETATE DE DI-GLYCYLGLYCYL N,N'-CYSTAMINE : I 116**

Réaction effectuée selon la méthode genérale déjà décrite.

Réactifs utilisés 2,3 g (3 9 x $10^{-3}$ mole) de di-Boc-di-glycylglycyl-N,N'-cystamine. 6 ml de TFA.

Au bout de 2 heures d'agitation à température ambiante, on ne distingue plus de produit de départ sur la CCM. On procède alors aux traitements déjà décrits et on recueille, après lyophilisation 2,22 g de produit sous forme huileuse (Rdt = 93 6%).

Ce sel sera conservé sous atmosphère d'azote ou sous vide.

Spectre de RMN ($D_20$) δ ppm: 3, 95 et 3,86 (2S-8H) $CH_2$ gly: 3,51 (m-4H) N-$CH_2$; 2,93 (m-4H) S-$CH_2$.

**EXEMPLE 37**

**BOC-GLYCYL-[N-(MEHYL-2 ACETYLMERCAPTO-2 ETHYL)] AMIDE**

Une solution agitée de 6,8 g (25 x $10^{-3}$ mole) d'ester Boc-glycyl-O-N-succinimide dans 100 ml d'acéto-nitrile est refroidie à 0°C et additionnée de 4,23 g (25 x $10^{-3}$ mole) de chlorhydrate de méthyl-2 acétyl-mercapto-2 éthylamine puis goutte à goutte d'une solution de 3,48 ml (25 x $10^{-3}$ mole) de triéthylamine dans 10 ml d'acétonitrile. La température réactionnelle est maintenue a 0°C durant toute l'addition de la TEA puis on laisse le melange revenir à la température ambiante et on prolonge l'agitation durant 8 heures. La solution est alors évaporée à sec sous vide. Le résidu blanc est repris par du dichlorométhane et on procède aux differents lavages (eau, bicarbonate de sodium aqueux saturé et glacé eau acide citrique 10 % dans l'eau, eau) jusqu'à pH neutre. Les phases organiques sont rassemblées, séchées sur sulfate de sodium et évaporées à sec sous vide. On obtient 7,6 g de produit brut qui sera chromatographié sur colonne de silicagel en utilisant un éluant constitué d'un mélange de benzène et d'acétate d'éthyle v/v 7-3. On recueille après cristallisation dans un melange d'éther et d'hexane ou d'acétate d'éthyle et d'éther de pétrole 1,38 g de cristaux incolores CCM (Benzéne-acétate d'éthyle 5-5). RF = 0,3. Rdt = 19%. F = 70-73°C.

Analyse $C_{12}$ $H_{22}$ $N_2$ S $O_4$ (290)

Calculé % C 49,65 H 7,58 N 9,65

Trouvé % C 49 63 H 7,54 N 9,62

Spectre IR (KBr) ν $cm^{-1}$: 3360, 3320 (NH) 2990, 2923 (CH-$CH_2$); 1685, 1660 (C=O); 1525 (amide I).

Spectre de RMN ($CDCl_3$) δ ppm: 6,70 et 5,40 (2m-2H) 2NH; 3,78 (d-2H) $CH_2$ gly; 3,43 (m-3H) N-$CH_2$-CH; 2,33 (S-3H) $COCH_3$; 1,46 (S-9H) terbutyl; 1,29 (d-3H) $CH_3$.

**EXEMPLE 38**

**TRIFLUOROACETATE DE GLYCYL-N-[(METHYL-2 ACETYLMERCAPTO- 2 ETHYL)] AMIDE: I 118**

Méthode générale de déprotection d'un BOC (déjà décrite). Réactifs utilisés 1,38 g (47 x $10^{-3}$ mole) de BOC-glycyl-N-[(méthyl-2 acétylmercapto-2 éthyl)]-amide. 7 ml de TFA.

La réaction suivie par CCM jusqu'à disparition du BOC est totale en 2 heures. Le mélange réactionnel est ensuite évaporé à sec sous vide et l'huile obtenue est reprise par 50 ml d'eau distillée. La phase aqueuse lavée au dichlorométhane (3 x 50 ml) puis lyophilisée fournit 1,38 g de trifluoroacétate sous forme huileuse et hygroscopique. Rdt = 97,18%.

Spectre de RMN ($D_20$) δ ppm: 3,69 (S-2H) $CH_2$ gly; de 3,65 à 3,15 (2m-3H) N-$CH_2$-CH; 2,28 (S-3H) $COCH_3$; 1,20 (d-3H) $CH_3$.

**EXEMPLE 39**

**BOC-SARCOSYL-N-(S-ACETYL)-CYSTEAMINE**

Réaction entre la BOC-Sarcosine et la S-acétyl-cystéamine faisant intervenir un agent de couplage selon la méthode genérale déjà décrite.

Réactifs utilisés: 9,58 g (50,7 x $10^{-3}$ mole) de BOC-Sarcosine dans 70 ml d'acétonitrile. 8,82 g (25,3 x $10^{-3}$ mole) de t-PNC dans 90 ml d'acétonitrile. 7,08 ml (50,7 x $10^{-3}$ mole) de TEA. Le mélange est agité à 0°C durant 15 minutes et additionné de 8,97 g (57 x $10^{-3}$ mole) de chlorhydrate de S-acétyl-cystéamine et d'une solution de 10,86 ml (77 x $10^{-3}$ mole) de TEA dans 30 ml acétonitrile.

Le mélange réactionnel est agité 12 heures à température ambiante puis évaporé à sec sous vide. Le résidu est repris par 400 ml de dichlorométhane et on procède aux différents lavages (eau, bicarbonate de sodium aqueux saturé et glacéeau, acide citrique 10% dans l'eau, eau jusqu'à pH neutre). La phase organique séchée sur sulfate de sodium puis évaporée à sec sous vide fournit 11,9 g de produit brut qui sera chromatographié sur colonne de silicagel en utilisant um éluant constitué d'un mélange d'acétate d'éthyle contenant 20 % d'ether de pétrole. On recueille après cristallisation éthyle et d'éther de pétrole 5,52 g de cristaux incolores. CCM (Acétate d'éthyle contenant 20 % d'éther de pétrole). RF = 0 5. Rdt = 37,6 %. F = 77-79°C.

Analyse $C_{12} H_{22} N_2 S O_4$ (290)

Calculé % C 49,65 H 7,58 N 9,65

Trouvé % C 49,62 H 7,61 N 9,67

Spectre IR (KBr) ν $cm^{-1}$ = 3270 (NH); 3080,2980,2930 ($CH-CH_2$); 1700; 1655 (C=O); 1560 (Amide).

Spectre RMN ($CDCl_3$) δ ppm: 6,50 (m-1H) NH; 3,88 (S-2H) $CH_2$ gly; 3,47 (m-2H) N-$CH_2$; 3,04 (m-2H) S-$CH_2$; 2,93 (S-3H) N-$CH_3$; 2,36 (S-3H) $COCH_3$; 1,50 (S-9H) terbutyl.

**EXEMPLE 40**

**TRIFLUOROACETATE DE SARCOSYL-N-(S-ACETYL)-CYSTEAMINE : I 123**

Méthode générale de déprotection d'un BOC (déjà décrite).

Réactifs utilisée 2 g (6,8 x $10^{-3}$ mole) de BOC-Sarcosyl-N-(S-acétyl)-cysteamine. 7 ml de TFA. La réaction suivie par CM est complàte en 2 heures. Après les différents lavages on recueille 1,87 g de poudre incolore. Rdt = 89,6%. F = 91-93°C.

Spectre IR (KBr) ν $cm^{-1}$: plusieurs bandes indiquant la présence d'un sel d'amine, 3240, 3090, 2975, 2840, 2620, 2260; 1700, 1670 (C=0); 1580 (amide).

Spectre de RMN ($D_2O$) δ ppm: 3,83 (S-2H) $CH_2$ gly ; 3,47 (m-2H) N-$CH_2$; 3,06 (m-2H) S-$CH_2$; 2,75 (S-3H) N-$CH_3$; 2,39 (S-3R) $COCH_3$.

**EXEMPLE 41**

**DI-BOC-SARCOSYL-N,N'-CYSTAMINE**

Réaction effectuée selon la méthode générale déjà décrite qui fait intervenir un ester activé de la BOC-Sarcosine et la cystamine.

Réactifs utilisés: 3,15 g (14 x $10^{-3}$ mole) de dichlorhydrate de cystamine dans 50 ml de DMF et 3,9 ml de TEA. 7,15 g (25 x $10^{-3}$ mole) d'ester BOC-Sarcosyl-O-N-Succinimide.

Le mélange réactionnel est agité 12 heures a température ambiante puis évaporé à sec sous vide. La pâte restante est reprise par 200 ml d'eau distillée et le précipité blanc formé est essoré puis cristallisé dans un mélange d'acétate d'éthyle de méthanol et d'éther de pétrole. On recueille 4,5 g du composé attendu. Rdt = 65 %. F = 178-180°C. CCM (dichlorométhane contenant 5% de méthanol). RF = 0,3.

Analyse $C_{20} H_{38} N_4 S_2 O_6$ (494)

Calculé %: C 48,58 H 7,69 N 11,33

Trouvé %: C 48,54 H 7,66 N 11,35

Spectre IR (KBr) ν $cm^{-1}$: 3270 (NH); 3080, 2980, 2930 ($CH-CH_2$); 1700, 1660 (C=O); 1560 (Amide).

Spectre RMN (DMSO) δ ppm: 8,15 (t-2H) NH; 3,84 (S-4H) $CH_2$ gly; 3,42 (m-4H) N-$CH_2$; 2,86 (m-4H) S-$CH_2$; 2,86 (S-6H) $CH_3$; 1,42 (S-18H) terbutyl.

**EXEMPLE 42**

**DI-TRIFLUOROACETATE DE DI-SARCOSYL-N,N'-CYSTAMINE: I 124**

Méthode générale de déprotection d'un BOC (déjà décrite). Réactifs utilisés: 2 g (4 x $10^{-3}$ mole) de Di-BOC-sarcosyl-N,N'-cystamine. 7 ml de TFA. La réaction suivie par CCM est complète en 1 heure. Après les différents lavages on recueille 1,96 g de poudre incolore et hygroscopique. Rdt = 93 8%.

Spectre de RM ($D_2O$) δ ppm: 3,83 (S-4H) $CH_2$ gly; 3,52 (t-4H) N-$CH_2$; 2,83 (t-4H) S-$CH_2$; 2,75 (S-6H) $CH_3$.

**EXEMPLE 43**

**ACIDE (GLYCYLAMINO-2)-ETHYLTHIOSULFURIQUE : I 125**

Une solution, contenant 1,31 g (5 x $10^{-3}$ mole) de bromhydrate de glycyl[N-(bromo-2-éthyl)]-amide 1,24 g (5 x $10^{-3}$ mole) de thiosulfate de sodium 5 $H_2O$ 0,68 g (5 x $10^{-3}$ mole) d'acétate de sodium 3 $H_2O$, et 6 ml d'eau

distillée est chauffée à 90-95°C jusqu'à disparition du bromure de départ. La réaction est suivie par CCM (butanol-éthanol-eau v.v.v : 2-1-1). RF du bromure 0,7. (Dans le même éluant RF du composé attendu 0,15). Le temps réactionnel est d'environ 2 heures. Par addition d'éthanol il y a apparition d'une huile. Le surnageant est décanté, l'huile est redissoute dans le minimum d'eau distillée et à nouveau on additionne de l'éthanol. Cette opération de lavage de l'huile est répétée 5 fois. La phase huileuse obtenue est alors reprise par de l'eau distillée et décolorée par du noir animal. La solution incolore est ensuite lyophilisée et fournit une poudre hygroscopique qui contient encore du thiosulfate de sodium.

Le produit pur sera isolé après filtration sur colonne de silicagel en utilisant un gradiant d'éluants constitué d'acétonitrile et d'eau. Rdt = 40 %. Ce composé sera conservé sous atmosphère d'azote.

Spectre de RMN ($D_2O$) 3,85 (S-2H) $CH_2$ gly; 3,63 (m-2H) N-$CH_2$: 3,25 (m-2H) $SCH_2$.

## EXEMPLE 44

### GLYCYLAMINO-2 S-ETHYLPHOSPHOROTHIOATE DE SODIUM : I 126

Dans 9,4 ml d'eau agitée à 15-20°C on additionne en maintenant cette température 1,714 g (9,52 x $10^{-3}$ mole) de phosphorothioate de sodium *. On obtient une suspension blanche. On ajoute ensuite 2,5 g (9,54 x $10^{-3}$ mole) de bromhydrate de glycyl[N-(bromo-2 éthyl)]-amide. Lorsque le mélange est devenu homogène (1 heure), on additionne goutte à goutte, en maintenant toujours la temperature entre 15 et 20°C, 4,7 ml de DMF. L'agitation à température ambiante est poursuivie durant 1 h 30 puis la solution est versée sur 60 ml de méthanol et placée au réfrigérateur durant 12 heures. Le précipité formé est essoré, repris par 10 ml d'eau et versé sur 60 ml de méthanol. La solution est maintenue à 0°C durant 2 heures et les cristaux du dérivé monosodé de l'acide S-(glycylamino-2) éthyl phosphorothioïque sont essorés et séchés sous vide dans un dessicateur contenant de l'anhydre phosphorique. On recueille environ 1,4 g de sel très hygroscopique.

Spectre de RMN ($D_2O$) $\delta$ ppm: 3,86 (S-2H) $CH_2$ gly; 3,40 et 2,80 (2m-4H) N-$CH_2$-$CH_2$-S.

* Le phosphorothioate trisodé est préparé selon la méthode de S. AKERFELOT; Acta. Chem. Scand., 1960, 14, 1980-84.

## EXEMPLE 45

### DI-TRIFLUOROACETATE DE DI-ALANYL-N,N'CYSTAMINE

La réaction est effectuée comme aux exemples 17 et 18 en remplaçant la glycine par l'alanine, les proportions molaires restant les mêmes. Ce produit se présente après lyophilisation sous forme d'une huile.

Spectre de RMN ($D_2O$), 3,98 (m) CH; 3,48 (m) N-$CH_2$; 2,76(t) S-$CH_2$; 1,46 (d) $CH_3$.

## EXEMPLE 46

### ACTIVITE RADIOPROTECTRICE

Cette étude d'activite radioprotectrice a été realisée selon une technique décrite par M. Fatome et coll., Eur. J. Med. Chem., 1977, 12, (1), 93. Les substances ont été injectées par voie intra-péritonéale aux doses indiquées sur le tableau I, 15 minutes avant une irradiation à la $DL_{100}$/30 jours. Cette dose étant égale à 950 Rad.

Des études plus complètes sont rapportées dans le tableau II.

TABLEU I

Taux de survie au 30ème jour
(injection des drogues 15' avant irradiation à 900 ou 950 Rad)

| Composé de l'exemple | $DL_{50}$ (mg/kg) | Doses (mg/kg i.p.) | % de survie |
|---|---|---|---|
| 2 | 1 500 | 750 | 100 |
| | | 187 | 67 |
| 4 | 1 500 | 750 | 80 |
| | | 188 | 40 |
| 6 | 800 | 400 | 60 |
| 8 | 1 200 | 750 | 93 |
| 10 | 800 | 800 | 40 |
| 16 | 1 500 | 750 | 100 |
| 18 | 1 200 | 750 | 100 |
| 19 | 800 | 400 | 100 |
| 45 | 1 200 | 600 | 100 |
| 22 | 1 250 | 625 | 100 |
| 24 | 1 500 | 750 | 70 |
| 26 | 1 200 | 750 | 60 |
| 28 | > 2 000 | 1 000 | 100 |
| 31 | 750 | 375 | 60 |
| 32 | 1 500 | 1 000 | 100 |
| 34 | 1 000 | 500 | 70 |
| 36 | 2 000 | 1 000 | 30 |

TABLEAU II

| Composé de l'exemple | Formules | DL 50 mg/kg (i.p.) | Doses administrées mg/kg (i.p.) | Temps d'administration avant irradiation | Doses de rayonnement (Rad.) | % de survie à 30 jours |
|---|---|---|---|---|---|---|
| 8 | TFA,$H_2N-CH_2-CO-NH-CH_2-CH_2-S-COCH_3$ | 1200 | 750<br>187<br>750 | 15'<br>15'<br>15' | 900<br>900<br>1100 | 93<br>0<br>50 |
| 18 | TFA,$H_2N-CH_2-CO-NH-CH_2-CH_2-S$<br>\|<br>TFA,$H_2N-CH_2-CO-NH-CH_2-CH_2-S$ | 1250 | 750<br>187<br>750<br>750<br>1000<br>750<br>187<br>94<br>47 | 15'<br>15'<br>2 heures<br>15'<br>15'<br>15'<br>15'<br>15'<br>15' | 900<br>900<br>900<br>1100<br>1300<br>1300<br>1100<br>900<br>900 | 100<br>90<br>60<br>100<br>0<br>10<br>0<br>30<br>0 |
| | TFA,$H_2N-CH(CH_3)-CO-NH-CH_2-CH_2-S$<br>\|<br>TFA,$H_2N-CH(CH_3)-CO-NH-CH_2-CH_2-S$ | 1200 | 600<br>150<br>600<br>600 | 15'<br>15'<br>2 heures<br>15' | 900<br>900<br>900<br>1100 | 100<br>20<br>50<br>50 |

**Tableau II** (Suite)

| N° de l'ex. | Formules | DL 50 mg/kg (i.p.) | Doses administrées mg/kg (i.p.) | Temps d'administration avant irradiation | Doses de rayonnement (Rad) | % de survie à 30 jours |
|---|---|---|---|---|---|---|
| 22 | TFA,$H_2N$-CH-CO-NH-$CH_2$-$CH_2$-S-$COCH_3$<br>\|<br>$CH_3$ | 1250 | 625<br>625<br>625<br>156 | 15'<br>15'<br>2 heures<br>15' | 900<br>1100<br>900<br>900 | 100<br>50<br>10<br>0 |
| 24 | $CH_3$<br>\|<br>TFA,$H_2N$-$CH_2$-CO-S-$CH_2$-C-NH-$COCH_3$<br>\|<br>$CH_3$ | 1500 | 750<br>750<br>750 | 15'<br>2 heures<br>15' | 900<br>900<br>1100 | 70<br>50<br>13 |
| 26 | $CH_3$<br>\|<br>TFA,$H_2N$-$CH_2$-CO-NH-C-$CH_2$-S-$COCH_3$<br>\|<br>$CH_3$ | 1200 | 750<br>187<br>750<br>750 | 15'<br>15'<br>2 heures<br>15' | 900<br>900<br>900<br>1100 | 60<br>40<br>70<br>0 |
| 28 | TFA,$H_2N$-$CH_2$-CO-NH-$CH_2$-CO-NH$(CH_2)_2$S-CO<br>\|<br>$CH_3$ | >2000 | 1000<br>1000<br>1500 | 15'<br>15'<br>15' | 900<br>1100<br>1100 | 100<br>0<br>100 |

**Tableau II** (Suite)

| N° de l'ex. | Formules | DL 50 mg/kg (i.p.) | Doses administrées mg/kg (i.p.) | Temps d'administration avant irradiation | Doses de rayonnement (Rad) | % de survie à 30 jours |
|---|---|---|---|---|---|---|
| 31 | TFA, $H_2N-CH_2-CO-NH-CH_2-CH-CH_2-S$<br>$OCOCH_3$ \|<br>TFA, $H_2N-CH_2-CO-NH-CH_2-CH-CH_2-S$<br>$OCOCH_3$ | 750 | 375<br>93,7<br>375 | 15'<br>15'<br>2 heures | 900<br>900<br>900 | 60<br>0<br>0 |
| 32 | TFA, $H_2N-CH_2-CO-NH-CH_2-CH-CH_2-S-CO$<br>$OCOCH_3$ $CH_3$ | 1500 | 1000<br>250<br>1000 | 15'<br>15'<br>15' | 900<br>900<br>1100 | 100<br>70<br>80 |
| 34 | TFA, $H_2N-CH_2-CO-S-CH_2-CH_2-NH-COCH_3$ | 1000 | 500<br>125 | 15'<br>15' | 950<br>950 | 70<br>20 |
| 36 | TFA, $H_2N-CH_2-CO-NH-CH_2-CO-NH-CH_2-CH_2-S$<br>TFA, $H_2N-CH_2-CO-NH-CH_2-CO-NH-CH_2-CH_2-S$ | 2000 | 1000<br>250 | 15'<br>15' | 950<br>950 | 30<br>10 |

Plusieurs remarques peuvent être faites sur ces résultats.

Tout d'abord, les deux homologues supérieurs I 106 (exemple 22) et I 105 (exemple 45) des I 102 (exemple 8) et I 103 (exemple 18) décrits présentent une bonne radioprotection pour une faible toxicité. Cependant, le remplacement de la glycine par la L-alanine n'apporte pas de granda changements quant à l'activite radioprotectrice, l'amino-acide le meilleur restant toutefois la glycine.

Les dérivés I 107 (exemple 24) et I 108 (exemple 28) comportent une glycine pour les raisons qui ont été invoquées et une cystéamine diméthylée en position α de l'atome d'azote choisie par analogie avec un thiosulfate décrit par Piper et coll;

$H_2N\text{-}C(CH_3)_2\text{-}CH_2\text{-}S\text{-}SO_3H$ (J.R. Piper et coll., J. Med. Chem. 1966, 9, 911), composé présentant une bonne protection à 600 mg/kg. Ces deux produits, acetylés en bout de chaîne, montrent une activité radioprotectrice ceci était prévisible pour le I 108 mais un fait nouveau est apparu car le I 107 est un S-glycyl au lieu d'un S-acetyle et cette fois l'amine terminale de la cystéamine comporte le groupement acétyle. L'activité étant liée à la libération in vivo du thiol, il doit donc y avoir dans ce cas une libération du groupement glycyl.

La molécule I 109 (exemple 28) est particulièrement intéressante. C'est la première à avoir une séquence dipeptidique sur la cystéamine S-acétylée. En utilisant un dipeptide (gly-gly) on souhaitait diminuer la toxicité par rapport au I 102 tout en gardant une activité radioprotectrice.

Les premiers résultats obtenus son très encourageants puisque la DL 50 du I 109 est >2000 mg/kg. Son activité, qui demande à être encore étudiée, est assez remarquable puisque l'on constate une survie de 100% à 30 jours sur des souris ayant reçu 1 500 mg/kg (i.p.) de drogue 15' avant une irradiation de 1 100 Rad.

Les compoeés I 110 (exemple 31) et I 111 (exemple 32) ont été synthétisée par analogie avec le WR 77913 ($H_2N\text{-}CH_2\text{-}CH(OH)\text{-}CH_2\text{-}S\text{-}PO_3HNa$) qui donne 100 % de survie a 30 jours sur des souris ayant reçu 40mg/kg (i.p.) 30' avant une irradiation de 950; 970 Rad. (J.R. Piper et coll., J. Med. Chem. 1975, 18, 804).

Les résultats sont encore partiels; néanmoins le I 111 peu toxique (DL 50 >1 500) présente des survies à 30 jours de 80% pour 1 000 mg/kg (1 100 Rad) et de 70% pour 250 mg/kg (900 Rad).

Quant au dérivé I 114 (exemple 34), il présente une conjugaison S-glycyl (voir le I 107) et peut être considéré comme l'inverse du I 102, il présente une légère action radioprotectrice.

**EXEMPLE 47**

**ACTIVITE ANTI-CARCINOGENE**

Droge utilisée:

HBr, $H_2N\text{-}CH_2\text{-}(C{=}O)\text{-}NH\text{-}(CH_2)_2\text{-}S\text{-}(C{=}O)\text{-}CH_3$ (I 102)

L'activité anti-carcinogène a été déterminée sur des souris porteuses de tumeurs EMT6 ou HT19. On injecte par voie i.p. 695 mg/kg 15 minutes avant le début de l'irradiation à 20 Gy.

Dans le premier cas, on observe pour la dose de 20 Gy une survie de $10^{-2}$, mais avec l'administration de la drogue la survie cellulaire est comprise entre 5 et $8.10^{-2}$ (selon que l'on tient compte ou non du coefficient de clonage).

Pour le second type de tumeur, on observe pour la dose de 20 Gy une survie de $10^{-2}$ mais avec administration de la drogue la survie cellulaire est comprise entre 2,4 et $4.10^{-2}$ (même remarque).

On constate donc que ce composé, outre son pouvoir radioprotecteur ne protège pas (ou peu) les tumeurs, sauf si celles-ci sont necrosées.

L'un des meilleurs radioprotecteurs actuels, le WR 2721, est structurellement proche du I 102 puisqu'il contient une cystéamine conjuguée avec un groupement propylamine et a sa fonction thiol protégée par un groupement phosphorothioate (instable d'ailleurs, d'où les problèmes rencontrés pour purifier le WR 2721). De plus il a été montré, principalement par les travaux de Yuhas que le WR 2721 présentait la propriété remarquable de se concentrer sélectivement dans les tissus normaux (et non dans les cellules cancéreuses).

La conséquence est qu'il protège sélectivement les tissus normaux contre les dommages induits par les radiations, tout en laissant les tumeurs solides supporter seules l'effet des radiations.

Or, dans la mesure où le nombre de cancers induits par la radiothérapie n'est pas négligeable, il y aurait donc là une drogue de choix pouvant être utilisée d'une manière très générale lors de tous les traitements du cancer par cette approche.

De plus, il semblerait aussi que le WR 2721 puisse exercer un effet protecteur sélectif lors des chimiothérapies effectuées avec les drogues alkylantes.

On a montré récemment que le composé type I 102 présentait cette même sélectivité.

Bien que le composé type de départ (I 102) présente un FRD moindre que le WR 2721 (respectivement 1.4 et 2.7 sur la moelle osseuse), le facteur de gain (FRD moelle osseuse/FRD tumeurs) pour un niveau de survie de $5.10^{-1}$ sur les tumeurs EMT6, est identique pour les deux produits et égal à 1.2.

De plus, le I 102 montre deux avantages par rapport au WR 2721:

- une plus grande stabilité chimique (le WR 2721 en effet n'est pas très stable, ce qui conduit parfois à des résultats non reproductibles),
- une toxicité moindre (DL 50 respective > 1 500 mg/kg et 950 mg/kg).

## ABREVATIONS UTILISEES DANS LA PARTIE EXPERIMENTALE

- Z: benzyloxycarbonyl
- t-PNC: hexachlorocyclotriphosphazène
- THF: tétrahydrofuranne
- TEA: triéthylamine
- CCM: chromatographie sur couche mince (Kieselgel Merck 60F 254)
- BOC: ter-butoxycarbonyl
- AA: amino-acide ou un radical correspondant à $R_1$-CO
- TFA: acide trifluoroacétique
- AcOEt: acétate d'éthyle
- Spectre de RMN: s = singulet, d = doublet, t = triplet, m = multiplet
- Produits cormerciaux de provenance Fluka
- MEA: β -mercapto-éthylamine (cystéamine)
- Ø: température
- DMF: diméthylforamide
- MEA: $NH_2$-$CH_2$-$CH_2$-SH ou le radical correspondant
- Chromatographies sur colonnes réalisées avec Kieselgel Merck 60 (70,230 Mesch).

## Revendications

1) Composé de formule I:

$R_1$-CO-$NR_3$-/A/-S-$R_2$

dans laquelle $R_1$ est un radical correspondant à un aminoacide $R_1$-COOH, /A/ est un radical alkylène en $C_2$ ou $C_3$ qui peut être substitué par un radical alcoyle en $C_1$ à $C_3$, un radical hydroxy, hydroxycarbonyl ou un radical -COR où R est un radical amino ou -NH-$CH_2$-$CO_2$H, $R_2$ est un radical protecteur de la fonction thiol qui libère la fonction thiol in vivo, $R_3$ est l'hydrogène ou relié au groupement $R_2$ forme un cycle protecteur de $R_2$ fonction thiol, à l'exeption de la diglycyl-N,N'cystamine, de la di-β-alanyl-N,N'-cystamine et de la di-phénylalanyl-N,N'cystamine, ainsi que les sels de ce composé avec des acides pharmaceutiquement acceptables.

2) Composé selon la revendication 1 dans lequel $R_1$ est un radical alcoylamino droit ou ramifié en $C_1$ à $C_7$.

3) Composé selon l'une des revendications 1 ou 2, dans lequel $R_2$ est le radical

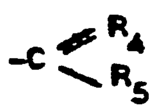

où $R_4$ = O, S, NH, N($C_1$-$C_7$)alkyl, $R_5$ = H, $C_1$-$C_7$-alkyl, aryl substitué ou non, $NH_2$, SH.

4) Composé selon l'une des revendications 1 ou 2, dans lequel $R_2$ est un radical -S-$R_6$, $R_6$ étant une chaîne $R_1$-CO-NH-/A/-
ou un radical aryl ou alkyl non substitué ou substitué, sous réserve que lorsque A est le radical éthylène, alors $R_1$COOH n'est pas la glycyne, la β-alanine ou la phénylalanine.

5) Composé selon l'une des revedications 1 ou 2, dans lequel $R_2$ est -$SO_3$H ou -$PO_3H_2$.

6) Composé selon l'une des revedication 1 ou 2, de formule:

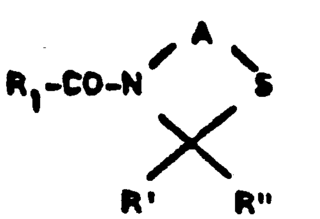 ou 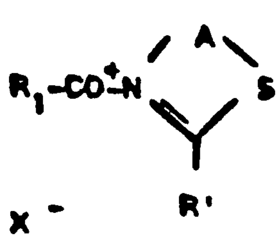

dans lesquelles R' = H, $C_1$-$C_7$-alkyl, aryl, $NH_2$, R'' = H, $C_1$-$C_7$-alkyl, aryl.

7) Composé selon l'une des revendications 1 à 6, sous forme d'une sel d'ammonium quaternaire, l'anion étant choisi parmi: $BR^-$, $Cl^-$, $CF_3CO_2^-$, $CH_3C_6H_4$-$SO_3^-$, $CH_3CO_2^-$.

8) Composé selon la revendication 1 de formule:
$NH_2$-$alk_1$-CO-NH-$alk_2$-S-CO-$alk_3$
dans laquelle $alk_1$ est une chaîne alkylène droite ou ramifiée en $C_1$ à $C_7$, $alk_2$ est un chaîne éthylényl ou propylényl portant un ou plusieurs radicaux méthyle ou hydroxy, $alk_3$ est un radical $C_1$-$C_7$-alkyl substitué par un ou plusieurs atomes de chlore.

9) Composé selon la revendication 8:
la glycyl-N-(S-acétyl)-cystéamine
la L-alanyl-N-(S-acéthyl)-cystéamine
la γ-aminobutyryl-N-(S-acétyl)-cystéamine
la glycyl-N-(S-dichloroacétyl)-cystéamine,
ainsi que les sels d'ammonium quaternaires correspondants.

10) Composé selon la revendication 1 de formule:

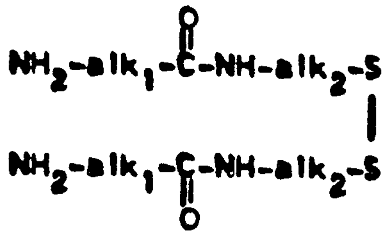

dans laquelle $alk_1$ est une chaîne alkylényl droite ou ramifiée en $C_1$ à $C_7$ et $alk_2$ est une chaîne éthylényl ou propylényl portant un ou plusieurs radicaux méthyle ou hydroxy, sous réserve que lorsque $alk_2$ est une chaîne éthylène, alors $alk_1$ n'est pas une chaîne éthylène ou méthylène.

11) Composé selon la revendication 10:
la di-γ-aminobutyryl-N,N'-cystamine,
ainsi que les sels d'ammonium quaternaires correspondants.

12) Procédé de préparation d'un composé selon l'une des revendications 1 à 11, caractérisé en ce qu'on déprotège un composé de formule:
(pro) $R_1$-CO-$NR_3$-/A/-S-$R_2$
en éliminant le ou les groupements protecteur pro du radical amino.

13) Procédé selon la revedication 12, caractérisé en ce que le groupement protecteur est le groupement benzyloxycarbonyl ou ter-butoxycarbonyl.

14) Application des composés selon l'une des revendications 1 à 11 ou de la diglycyl-N,N'cystamine, de la di-β-alanyl-N,N'-cystamine et di-phénylalanyl-N,N'-cystamine à titre d'agent radioprotecteur.

15) Compositon pharmaceutique comportant à titre de principe actif au moins un composé selon l'une des revendications 1 à 11 ou la diglycyl-N,N'cystamine, la di-β-alanyl-N,N'-cystamine et la di-phénylalanyl-N,N'cystamine.

**Patentansprüche**

1. Verbindung der Formel (I):
$R_1$-CO-$NR_3$-[A]-S-$R_2$
worin bedeuten:
$R_1$ einen Rest, der einer Aminosäure $R_1$-COOH entspricht,
[A] einen $C_2$- oder $C_3$-Alkylenrest, der substituiert sein kann durch einen $C_1$-$C_3$-Alkylrest, einen Hydroxyrest, einen Hydroxycarbonylrest oder einen Rest -COR, worin R einen Aminorest oder den Rest -NH-$CH_2$-COOH darstellt,
$R_2$ einen Thiolfunktion-Schutzrest, der die Thiolfunktion in vivo freisetzt,
$R_3$ Wasserstoff oder in Verbindung mit der Gruppe $R_2$ einen $R_2$-Schutzring für die Thiolfunktion,
mit Ausnahme von Diglycyl-N,N'-cystamin, Di-β-alanyl-N,N'-cystamin und Di-phenylalanyl-N,N'-cystamin, sowie die Salze dieser Verbindung mit pharmazeutisch akzeptablen Säuren.

2. Verbindung nach Anspruch 1, worin $R_1$ einen unverzweigten oder verzweigten $C_1$-$C_7$-Alkylaminorest bedeutet.

3. Verbindung nach Anspruch 1 oder 2, worin $R_2$ den Rest darstellt

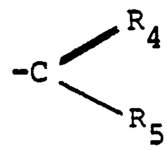

worin $R_4$ = O, S, NH, N-($C_1$-$C_7$)-Alkyl, $R_5$ = H, $C_1$-$C_7$-Alkyl, Aryl, substituiert oder nicht substituiert, $NH_2$, SH.

4. Verbindung nach Anspruch 1 oder 2, worin $R_2$ bedeutet einen Rest -S-$R_6$, worin $R_6$ darstellt eine Kette
$R_1$-CO-NH-[A]-
oder einen Aryl- oder Alkylrest, unsubstituiert oder substituiert, mit der Maßgabe, daß dann, wenn A den Ethylenrest bedeutet, $R_1$COOH nicht für Glycin, β-Alanin oder Phenylalanin steht.

5. Verbindung nach Anspruch 1 oder 2, worin $R_2$ -$SO_3H$ oder -$PO_3H_2$ bedeutet.

6. Verbindung nach Anspruch 1 oder 2 der Formel

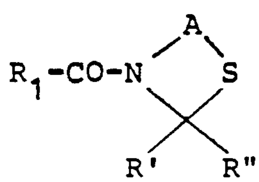

oder

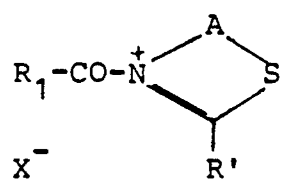

worin R' = H, $C_1$-$C_7$-Alkyl, Aryl, $NH_2$, R'' = H, $C_1$-$C_7$-Alkyl, Aryl.

7. Verbindung nach einem der Ansprüche 1 bis 6 in Form eines quaternären Ammoniumsalzes, dessen Anion ausgewählt wird aus $Br^-$, $Cl^-$, $CF_3CO_2^-$, $CH_3$-$C_6H_4$-$SO_3^-$, $CH_3CO_2^-$.

8. Verbindung nach Anspruch 1 der Formel
$NH_2$-$Alk_1$-CO-NH-$Alk_2$-S-CO-$Alk_3$
worin bedeuten:
$Alk_1$ eine gerade oder verzweigte $C_1$-$C_7$-Alkylenkette,
$Alk_2$ eine Ethylenyl-oder Propylenylkette, die einen oder mehrere Methyl- oder Hydroxyreste trägt,
$Alk_3$ einen $C_1$-$C_7$-Alkylrest, der durch ein oder mehr Chloratome substituiert ist.

9. Verbindung nach Anspruch 8:
Glycyl-N-(S-acetyl)-cysteamin
L-Alanyl-N-(S-acetyl)-cysteamin
γ-Aminobutyryl-N-(S-acetyl)-cysteamin
Glycyl-N-(S-dichloroacetyl)-cysteamin
sowie die entsprechenden quaternären Ammoniumsalze.

10. Verbindung nach Anspruch 1 der Formel:

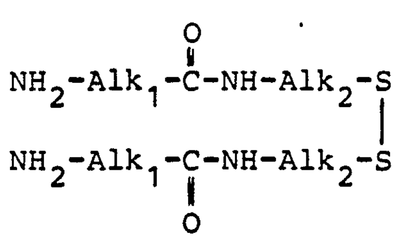

worin bedeuten:
$Alk_1$ eine unverzweigte oder verzweigte $C_1$-$C_7$-Alkylenylkette und

$Alk_2$ eine Ethylenyl- oder Propylenylkette, die ein oder mehrere Methyl- oder Hydroxyreste trägt, mit der Maßgabe, daß dann, wenn $Alk_2$ eine Ethylenkette darstellt, $Alk_1$ nicht eine Ethylen- oder Methylenkette bedeutet.

11. Verbindung nach Anspruch 10:
Di γ-aminobutyryl-N,N'-cystamin,
sowie die entsprechenden quaternären Ammoniumsalze.

12. Verfahren zur Herstellung einer Verbindung nach einem der Ansprüche 1 bis 11, dadurch gekennzeichnet, daß man eine Verbindung der Formel:
(pro) $R_1$-CO-$NR_3$-[A]-S-$R_2$
von der Schutzgruppe befreit, indem man die Schutzgruppe(n) (pro) des Aminorestes eliminiert.

13. Verfahren nach Anspruch 12, dadurch gekennzeichnet, daß es sich bei der Schutzgruppe um die Benzyloxycarbonyl- oder ter-Butoxycarbonylgruppe handelt.

14. Verwendung der Verbindungen nach einem der Ansprüche 1 bis 11 oder von Diglycyl-N,N'-cystamin, Di-β-alanyl-N,N'-cystamin und Di-phenylalanyl-N,N'-cystamin als Strahlenschutzmittel.

15. Pharmazeutische Zusammensetzung, die als Wirkstoff mindestens eine Verbindung nach einem der Ansprüche 1 bis 11 oder Diglycyl-N,N'-cystamin, Di-β-alanyl-N,N'-cystamin und Di-phenylalanyl-N,N'-cystamin enthält.

## Claims

1. A compound of formula I:
$R_1$-CO-$NR_3$-/A/-S-$R_2$
wherein $R_1$ is a radical corresponding to an amino acid $R_1$-COOH, /A/ is an alkylene radical in $C_2$ or $C_3$ which may be substituted by an alkyl radical in $C_1$ to $C_3$, a hydroxy, hydroxy carbonyl radical or a -COR radical where R is an amino radical or -NH-$CH_2$-$CO_2H$, $R_2$ is a radical protecting the thiol function which releases the thiol function in vivo, $R_3$ is hydrogen or bound with the $R_2$ group forms a cycle protecting $R_2$ thiol function, with the exception of diglycyl-N,N'cystamine, of di-β-alanyl-N,N'-cystamine and of di-phenylalanyl-N,N'-cystamine, as well as the salts of said compound with pharmaceutically acceptable acids.

2. A compound according to Claim 1 wherein $R_1$ is a straight or branched alkyl amino radical in $C_1$ to $C_7$.

3. A compound according to either of Claims 1 or 2, wherein $R_2$ is the radical

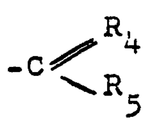

wherein $R_4$ = O, S, NH, N($C_1$-$C_7$) alkyl, $R_5$ = H, $C_1$-$C_7$-alkyl, aryl substituted or non-substituted, $NH_2$, SH.

4. A compound according to either of Claims 1 or 2, wherein $R_2$ is a -S-$R_6$ radical, $R_6$ being a chain
$R_1$-CO-NH-/A/-
or an aryl or alkyl radical, substituted or non-substituted, subject to the fact that when A is the ethylene radical, then $R_1$COOH is not glycin, β-alanine or phenyl alanine.

5. A compound according to either of Claims 1 or 2, wherein $R_2$ is -$SO_3H$ or -$PO_3H_2$.

6. A compound according to either of Claims 1 or 2, of formula:

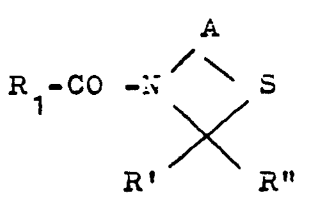

or

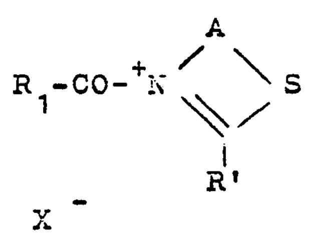

wherein R' = H, $C_1$-$C_7$-alkyl, aryl, $NH_2$, R'' = H, $C_1$-$C_7$ alkyl, aryl.

7. A compound according to one of Claims 1 to 6, in the form of a quaternary ammonium salt, the anion

having been selected from: $Br^-$, $Cl^-$, $CF_3CO_2^-$, $CH_3$-$C_6H_4$-$SO_3^-$, $CH_3CO_2^-$.

8. A compound according to Claim 1 of formula:

$NH_2$-$alk_1$-CO-NH-$alk_2$-S-CO-$alk_3$

wherein $alk_1$ is a straight or branched alkylene chain in $C_1$ to $C_7$, $alk_2$ is an ethylenyl or propylenyl chain bearing one or more methyl or hydroxy radicals, alk is a $C_1$-$C_7$ radical substituted by one or more chlorine atoms.

9) A compound according to Claim 8:

the glycyl-N-(S-acetyl)-cysteamine

the L-alanyl-N-(S-acetyl)-cysteamine

the γ-aminobutyryl-N-(S-acetyl)-cysteamine

the glycyl-N-(S-dichloroacetyl)-cysteamine

as well as the corresponding quaternary ammonium salts.

10. A compound according to Claim 1 of formula:

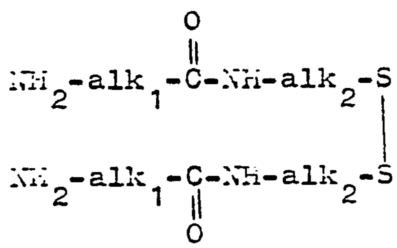

wherein $alk_1$ is a straight or branched alkenyl chain in $C_1$ to $C_7$ and alk is an ethylenyl or propylenyl chain bearing one or more methyl or hydroxy radicals, with the proviso that when $alk_2$ is an ethylene chain, then $alk_1$ is not an ethylene or methylene chain.

11. A compound according to Claim 10:

the di-γ-aminobutyl-N,N'-cystamine,

as well as the corresponding quaternary ammonium salts.

12. A method of preparation of a compound according to one of Claims 1 to 11, characterised in the disprotection of a compound of formula:

(pro) $R_1$-CO-$NR_3$-/A/-S-$R_2$

by eliminating the pro protective group or groups of the amino radical.

13. A method according to Claim 12, characterised in that the protective group is the benzyloxy-carbonyl or ter-butoxy carbonyl group.

14. Application of the compounds according to one of Claims 1 to 11 or of diglycyl-N,N'-cystamine, di-β-alanyl-N,N'-cystamine and di-phenylalanyl-N,N'-cystamine as radio-protector agent.

15. A pharmaceutical composition comprising as the active agent at least one compound according to one of Claims 1 to 11 or diglycyl-N,N'-cystamine, di-β-alanyl-N,N'-cystamine and di-phenylalanyl-N,N'-cystamine.